# EUROPÄISCHE PATENTANMELDUNG

(11) **EP 0 861 879 A2**
(43) Veröffentlichungstag der Anmeldung: **02.09.1998**
(21) Anmeldenummer: 97119151.5
(22) Anmeldetag: 03.11.1997
(51) Int. Cl.: C09B 62/513, C09B 35/52, C07C 311/41

(54) **Polyazofarbstoffe**

(30) Priorität: 26.11.1996 DE 19648939
(71) Anmelder: BASF AKTIENGESELLSCHAFT, 67056 Ludwigshafen (DE)
(72) Erfinder: Lamm, Gunther Dr., 67454 Hassloch (DE); Wiesenfeldt, Matthias Dr., 67125 Dannstadt-Schauernheim (DE)

(57) **Zusammenfassung**

Polyazofarbstoffe der Formel I 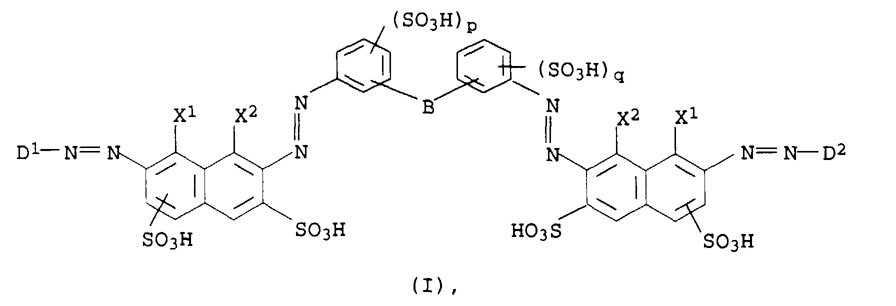 in der einer der beiden Reste X¹ und X² Hydroxy und der andere Amino,
p und q unabhängig voneinander eine Zahl 0 oder 1,
D¹ und D² unabhängig voneinander einen Rest der Formel 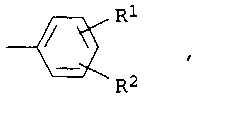
und B ein Brückenglied bedeuten, Sulfonamide als deren Zwischenprodukte sowie die Verwendung der Farbstoffe zum Färben von natürlichen oder synthetischen Substraten.

## Beschreibung

Die vorliegende Erfindung betrifft Polyazofarbstoffe der allgemeinen Formel I 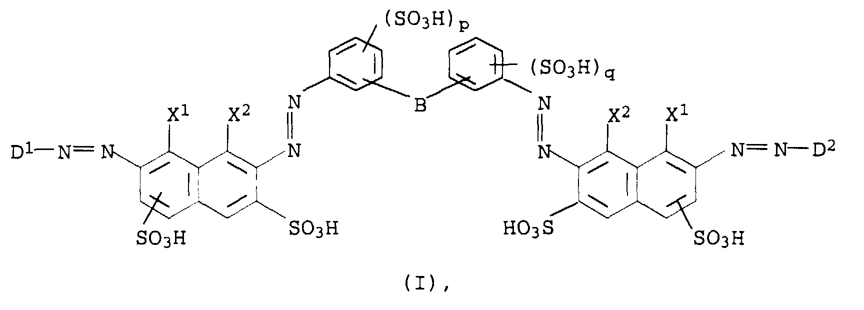 in der einer der beiden Reste X¹ und X² Hydroxy und der andere Amino,
- p: die Zahl 0 oder 1,
- q: die Zahl 0 oder 1,
- D¹ und D²: unabhängig voneinander einen Rest der Formel 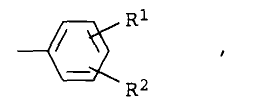
- B: ein Brückenglied der Formel
-SO₂-NH-A¹-NH-SO₂-, -SO₂-N(Alk)-A¹-NH-SO₂-, -SO₂-N(Alk)-A¹-N(Alk)-SO₂-, -SO₂-N(Alk)-A¹-O-SO₂-, -SO₂-O-A¹-O-SO₂-, -SO₂-N(Alk)-, -SO₂-N(Alk)-SO₂-, -SO₂-NH-A²-NH-SO₂-, -SO₃- oder SO₂ bedeuten, wobei
- A¹: für Phenylen, das mit Hydroxysulfonyl substituiert sein kann
- A²: für C₁-C₈-Alkylen
- R¹: für Wasserstoff, C₁-C₄-Alkyl, Halogen, Cyano, Nitro, Hydroxysulfonyl, Pyrrolidinylsulfonyl, Piperidinylsulfonyl, Morpholinylsulfonyl oder einen Rest der Formel CO-Ar, CO-OAr, CO-Alk, CO-OAlk, CO-N(Ar)Alk, CO-N(Alk)₂, SO₂-Ar, SO₂-Alk, SO₂-CH₂CH=CH₂, SO₂-CH=CH₂, SO₂-C₂H₄-Q, SO₂-OAr, SO₂-N(Alk)₂, SO₂-NHAlk, SO₂-N(Ar)Alk, SO₂-NHAr, 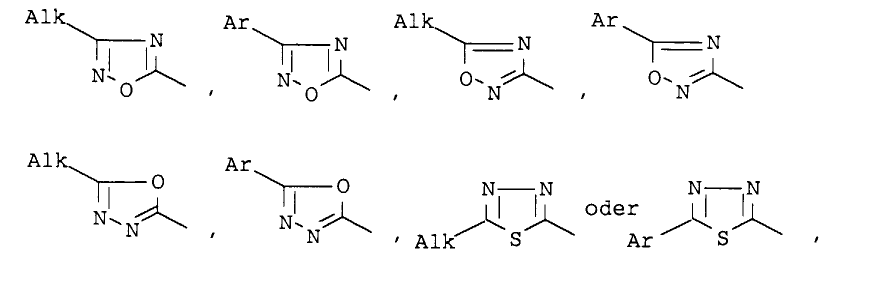
- Alk: für C₁-C₈-Alkyl, das durch 1 bis 3 Sauerstoffatome in Etherfunktion oder durch 1 Schwefelatom oder 1 Sulfonylgruppe unterbrochen sein kann und durch Hydroxy, C₁-C₄-Alkanoyloxy, Benzoyloxy, Sulfato, Halogen, Cyano, Carbamoyl, Carboxy, Hydroxysulfonyl, Phenyl oder Hydroxysulfonylphenyl substituiert sein kann, oder C₅-C₈-Cycloalkyl,
- Ar: für Phenyl oder Naphthyl, wobei diese Reste jeweils durch C₁-C₄-Alkyl, Halogen, C₁-C₄-Alkoxy, Phenoxy, Hydroxy, Carboxy, C₁-C₄-Alkanoylamino, dessen Alkylkette durch 1 Sauerstoffatom in Etherfunktion unterbrochen sein kann, Benzoylamino, Hydroxysulfonyl, Pyrrolidinylsulfonyl, Piperidinylsulfonyl, Morpholinylsulfonyl oder einen Rest der Formel SO₂-Alk, SO₂-CH₂CH=CH₂, SO₂-CH=CH₂, SO₂-C₂H₄-Q, SO₂-NHAlk, SO₂-N(Alk)₂, SO₂-G, SO₂-OG, SO₂-NHG oder SO₂-N(Alk)G substituiert sein können,
- R²: für Wasserstoff, Hydroxysulfonyl, C₁-C₄-Alkyl, C₁-C₄-Alkoxy oder einen Rest der Formel CO-Ar, CO-OAlk, CO-OAr, SO₂-Ar, SO₂-Alk, SO₂-OAr, 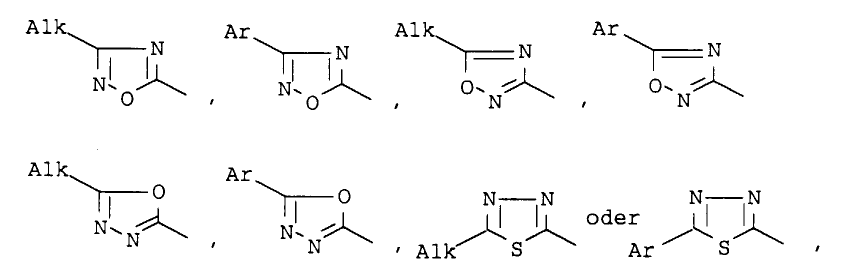
- Q: für Hydroxy oder eine unter alkalischen Reaktionsbedingungen abspaltbare Gruppe und
- G: für Phenyl, das durch C₁-C₄-Alkyl, Carboxyl, Hydroxysulfonyl oder C₁-C₄-Alkanoylamino substituiert sein kann, oder Naphthyl, das durch Hydroxysulfonyl substituiert sein kann, stehen,
Sulfonamide als deren Zwischenprodukte sowie die Verwendung der neuen Farbstoffe zum Färben von natürlichen oder synthetischen Substraten.

Die EP-A 716 130 beschreibt reaktivankertragende Azofarbstoffe aus 4-(β-Sulfatoethylsulfonyl)anilin und H-Säure, die über ihr Naphthalingerüst mit diazotiertem Bis(p-aminobenzolsulfon)imid oder diazotiertem p-Aminobenzolsulfon(p-aminophenyl)amid verdoppelt sind. An mit diesen Farbstoffen gefärbten Textilien sind jedoch durch Wäsche verursachte Farbumschläge zu beobachten.

Daher war es Aufgabe der vorliegenden Erfindung neue Polyazofarbstoffe bereitzustellen, die sich durch vorteilhafte anwendungstechnische Eigenschaften auszeichnen. Insbesondere sind darunter hohe Farbstärke in Verbindung mit guten Echtheiten zu verstehen.

Demgemäß wurden die eingangs näher bezeichneten Polyazofarbstoffe der Formel I gefunden.

Alle in der obengenannten Formel I auftretenden Alkylgruppen können sowohl geradkettig als auch verzweigt sein.

Wenn in der obengenannten Formel substituierte Alkylreste auf treten, so weisen sie in der Regel 1 oder 2 Substituenten auf.

Wenn in den obengenannten Formeln Alkylreste auftreten, die durch Sauerstoffatome in Etherfunktion unterbrochen sind, so sind solche Alkylreste bevorzugt, die durch 1 oder 2 Sauerstoffatome in Etherfunktion unterbrochen sind.

Wenn in den obengenannten Formeln substituierte Phenylreste auftreten, so weisen sie in der Regel 1 bis 3 Substituenten auf.

Wenn die Phenylengruppe substituiert ist, weist sie in der Regel 1 oder 2, bevorzugt einen Hydroxysulfonylrest auf.

Reste A¹ sind bevorzugt 1,2-, 1,3- und 1,4-Phenylen, 2-Hydroxysulfonyl-1,3-phenylen, 2-Hydroxysulfonyl-1,4-phenylen oder 2,5-Dihydroxysulfonyl-1,4-phenylen.

Reste A² und unten genannte Reste A³ sind z.B. CH₂, (CH₂)₂, (CH₂)₃, (CH₂)₄, CH(CH₃)CH₂, CH(CH₃)CH(CH₃), (CH₂)₅, (CH₂)₆, (CH₂)₇ und (CH₂)₈.

Reste R¹, R² und Alk sind z.B. Methyl, Ethyl, Propyl, Isopropyl, Butyl, Isobutyl, sec-Butyl oder tert-Butyl.

Reste R¹ und Halogen sind weiterhin z.B. Fluor, Chlor oder Brom.

Reste R² sind weiterhin z.B. Methoxy, Ethoxy, Propoxy, Isopropoxy, Butoxy, Isobutoxy, sec-Butoxy oder tert-Butoxy.

Reste Alk sind weiterhin z.B. Pentyl, Isopentyl, Neopentyl, tert-Pentyl, Hexyl, 2-Methylpentyl, Heptyl, 1-Ethylpentyl, Octyl, 2-Ethylhexyl, Isooctyl (Die obige Bezeichnung Isooctyl ist eine Trivialbezeichnung und stammt von den nach der Oxosynthese erhaltenen Alkoholen - vgl. dazu Ullmann's Encyclopedia of Industrial Chemistry, 5th Edition, Vol. A 1, Seiten 290 bis 293, sowie Vol. A 10, Seiten 284 und 285), 2-Methoxyethyl, 2-Ethoxyethyl, 2-Propoxyethyl, 2-Isopropoxyethyl, 2-Butoxyethyl, 2- oder 3-Methoxypropyl, 2- oder 3-Ethoxypropyl, 2- oder 3-Propoxypropyl, 2- oder 3-Butoxypropyl, 2- oder 4-Methoxybutyl, 2- oder 4-Ethoxybutyl, 2- oder 4-Propoxybutyl, 3,6-Dioxaheptyl, 3,6-Dioxaoctyl, 4,8-Dioxanonyl, 3,7-Dioxaoctyl, 3,7-Dioxanonyl, 4,7-Dioxaoctyl, 4,7-Dioxanonyl, 2- oder 4-Butoxybutyl, 4,8-Dioxadecyl, 3,6,9-Trioxadecyl, 3,6,9-Trioxaundecyl, 2-Methylthioethyl, 2-Ethylthioethyl, 2- oder 3-Methylthiopropyl, 2- oder 3-Ethylthiopropyl, 2- oder 4-Methylthiobutyl, 2- oder 4-Ethylthiobutyl, 2-Methylsulfonylethyl, 2-Ethylsulfonylethyl, 2- oder 3-Methylsulfonylpropyl, 2- oder 3-Ethylsulfonylpropyl, 2- oder 4-Methylsulfonylbutyl, 2- oder 4-Ethylsulfonylbutyl, Chlormethyl, 2-Chlorethyl, 2- oder 3-Chlorpropyl, Benzyl, 1- oder 2-Phenylethyl, 2-, 3- oder 4-Hydroxysulfonylbenzyl, 2-(2-,3- oder 4-Hydroxysulfonylphenyl)ethyl, 3-Benzyloxypropyl, 2-Hydroxyethyl, 2- oder 3-Hydroxypropyl, Cyanomethyl, 2-Cyanoethyl, 2- oder 3-Cyanopropyl, Carbamoylmethyl, 2-Carbamoylethyl, 2- oder 3-Carbamoylpropyl, 2-Acetyloxyethyl, 2- oder 3-Acetyloxypropyl, 2-Isobutyryloxyethyl, 2- oder 3-Isobutyryloxypropyl, Carboxylmethyl, 2-Carboxylethyl, 2- oder 3-Carboxylpropyl, 2-Hydroxysulfonylethyl, 2- oder 3-Hydroxysulfonylpropyl, 2-Sulfatoethyl, 2- oder 3-Sulfatopropyl, Cyclopentyl, Cyclohexyl, Cycloheptyl oder Cyclooctyl.

Reste Ar sind z.B. Phenyl, 2-, 3- oder 4-Methylphenyl, 2-, 3- oder 4-Ethylphenyl, 2-, 3- oder 4-Propylphenyl, 2-, 3- oder 4-Isopropylphenyl, 2-, 3- oder 4-Butylphenyl, 2,4-Dimethylphenyl, 2-, 3- oder 4-Phenoxyphenyl, 2-, 3- oder 4-Hydroxyphenyl, 2-, 3- oder 4-Methoxyphenyl, 2-, 3- oder 4-Ethoxyphenyl, 2-, 3- oder 4-Isobutoxyphenyl, 2,4-Dimethoxyphenyl, 2-, 3- oder 4-Formylaminophenyl, 2-, 3- oder 4-Acetylaminophenyl, 2-, 3- oder 4-Propionylaminophenyl, 2-, 3- oder 4-Methoxyacetylaminophenyl, 2-, 3- oder 4-Chlorphenyl, 2-, 3- oder 4-Carboxyphenyl, 2-, 3- oder 4-Hydroxysulfonylphenyl, 2-, 3- oder 4-Benzoylaminophenyl, 2-, 3- oder 4-Pyrrolidinylsulfonylphenyl, 2-, 3- oder 4-Piperidinsulfonylphenyl, 2-, 3- oder 4-Morpholinylsulfonylphenyl, 2-, 3- oder 4-Methylsulfonylphenyl, 2-, 3- oder 4-Carboxymethylsulfonylphenyl, 2-, 3- oder 4-Vinylsulfonylphenyl, 2-, 3- oder 4-(2-Hydroxyethyl)sulfonylphenyl, 2-, 3- oder 4-(2-Sufatoethyl)sulfonylphenyl, 2-, 3- oder 4-Ethylsulfamoylphenyl, 2-, 3- oder 4-(2-Hydroxyethyl)sulfamoylphenyl, 2-, 3- oder 4-Bis(2-hydroxyethyl)sulfamoylphenyl, Naphth-1-yl, Naphth-2-yl, 2-Hydroxysulfonylnaphth-1-yl, 5-Hydroxysulfonylnaphth-1-yl oder 5-Hydroxysulfonylnaphth-2-yl.

Der Rest Q steht für Hydroxy oder eine unter alkalischen Reaktionsbedingungen abspaltbare Gruppe. Solche Gruppen sind z.B. Chlor, Brom, C₁-C₄-Alkylsulfonyl, Phenylsulfonyl, OSO₃H, SSO₃H, OP(O)(OH)₂, C₁-C₄-Alkylsulfonyloxy, Phenylsulfonyloxy, C₁-C₄-Alkanoyloxy, C₁-C₄-Dialkylamino, Cyanamino oder ein Rest der Formel 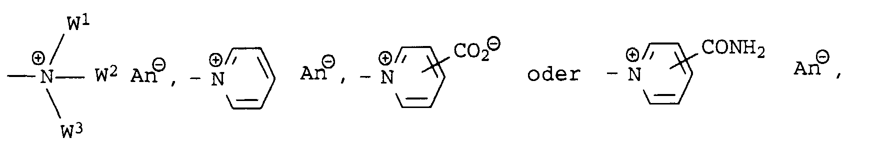 wobei W¹, W² und W³ unabhängig voneinander jeweils die Bedeutung von C₁-C₄-Alkyl oder Benzyl und An^{⊖} jeweils die Bedeutung eines Äquivalents eines Anions besitzen. Als Anionen können dabei z.B. Fluorid, Chlorid, Bromid, Iodid, Mono-, Di- oder Trichloracetat, Methylsulfonat, Phenylsulfonat oder 2- oder 4-Methylphenylsulfonat in Betracht kommen.

Rest R¹ und R² sind z.B. Benzoyl-, 2-, 3- oder 4-Methylbenzoyl, 2,3-, 2,4-, 2,5-, 2,6-, 3,4- oder 3,5-Dimethylbenzoyl, Methoxycarbonyl, Ethoxycarbonyl, Propoxycarbonyl, Isopropoxycarbonyl, Butoxycarbonyl, Methylsulfonyl, Ethylsulfonyl, Propylsulfonyl, Isopropylsulfonyl, Butylsulfonyl, 5-Methyl-, 5-Ethyl-, 5-Propyl-, 5-Butyl- oder 5-Phenyl-1,2,4-oxadiazol-3-yl, 5-Methyl-, 5-Ethyl-, 5-Propyl-, 5-Butyl oder 5-Phenyl-1,3,4-oxadiazol-2-yl, 3-Methyl-, 3-Ethyl-, 3-Propyl-, 3-Butyl- oder 3-Phenyl-1,2,4-oxadiazol-5-yl, Phenylsulfonyl, 2-, 3- oder 4-Methylphenylsulfonyl, 2,3-, 2,4-, 2,5-, 2,6-, 3,4- oder 3,5-Dimethylphenylsulfonyl, Phenoxycarbonyl, 2-, 3- oder 4-Methylphenoxycarbonyl, Phenoxysulfonyl oder 2-, 3- oder 4-Methylphenoxysulfonyl.

Reste R¹ sind weiterhin z.B. N-Phenyl-N-methylsulfamoyl, N-Phenyl-N-ethylsulfamoyl, N-Phenyl-N-propylsulfamoyl, N-Phenyl-N-butylsulfamoyl, Phenylsulfamoyl, 2-, 3- oder 4-Methylphenylsulfamoyl, Dimethylsulfamoyl, Diethylsulfamoyl, Dipropylsulfamoyl, Dibutylsulfamoyl, N-Methyl-N-ethylsulfamoyl, Bis(2-hydroxyethyl)sulfamoyl, Bis(carboxymethyl)sulfamoyl, Bis(2-carboxyethyl)sulfamoyl, Methylsulfamoyl, Ethylsulfamoyl, Propylsulfamoyl, Isopropylsulfamoyl, Butylsulfamoyl, 2-Hydroxyethylsulfamoyl, Carboxymethylsulfamoyl, 2-Carboxyethylsulfamoyl, Dimethylcarbamoyl, Diethylcarbamoyl, Dipropylcarbamoyl, Dibutylcarbamoyl, N-Methyl-N-ethylcarbamoyl, Bis(2-hydroxyethyl)carbamoyl, Bis(carboxymethyl)carbamoyl, Bis(2-carboxyethyl)carbamoyl, N-Phenyl-N-methylcarbamoyl, N-Phenyl-N-ethylcarbamoyl, N-Phenyl-N-propylcarbamoyl, N-Phenyl-N-butylcarbamoyl, 2-Hydroxyethylsulfonyl, 2-Chlorethylsulfonyl, 2-Sulfatoethylsulfonyl oder 2-Acetyloxyethylsulfonyl.

Die Reste D¹ und D² können innerhalb eines Moleküls verschieden sein und somit unterschiedliche Substituenten R¹ wie auch unterschiedliche Substituenten R² tragen.

Die Reste 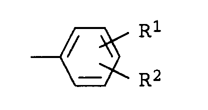 gehorchen z.B. den Formeln

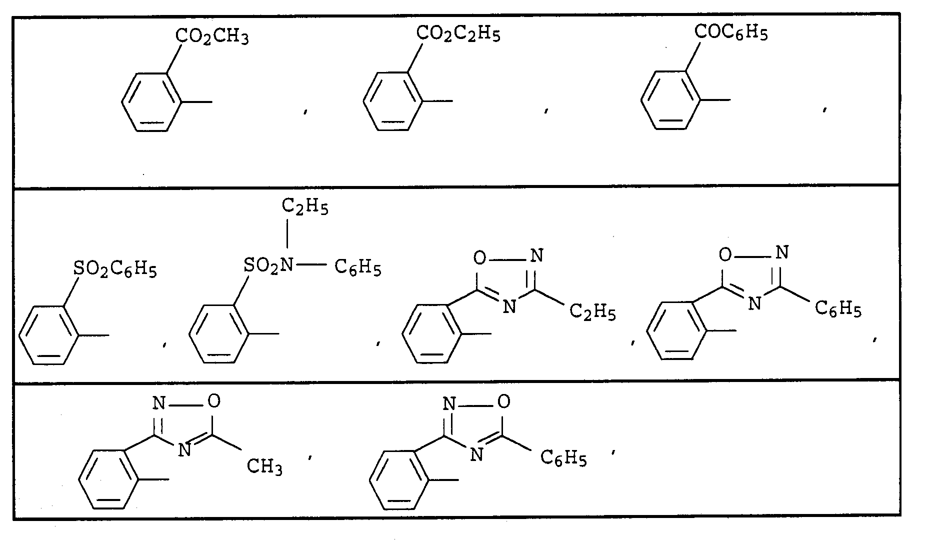

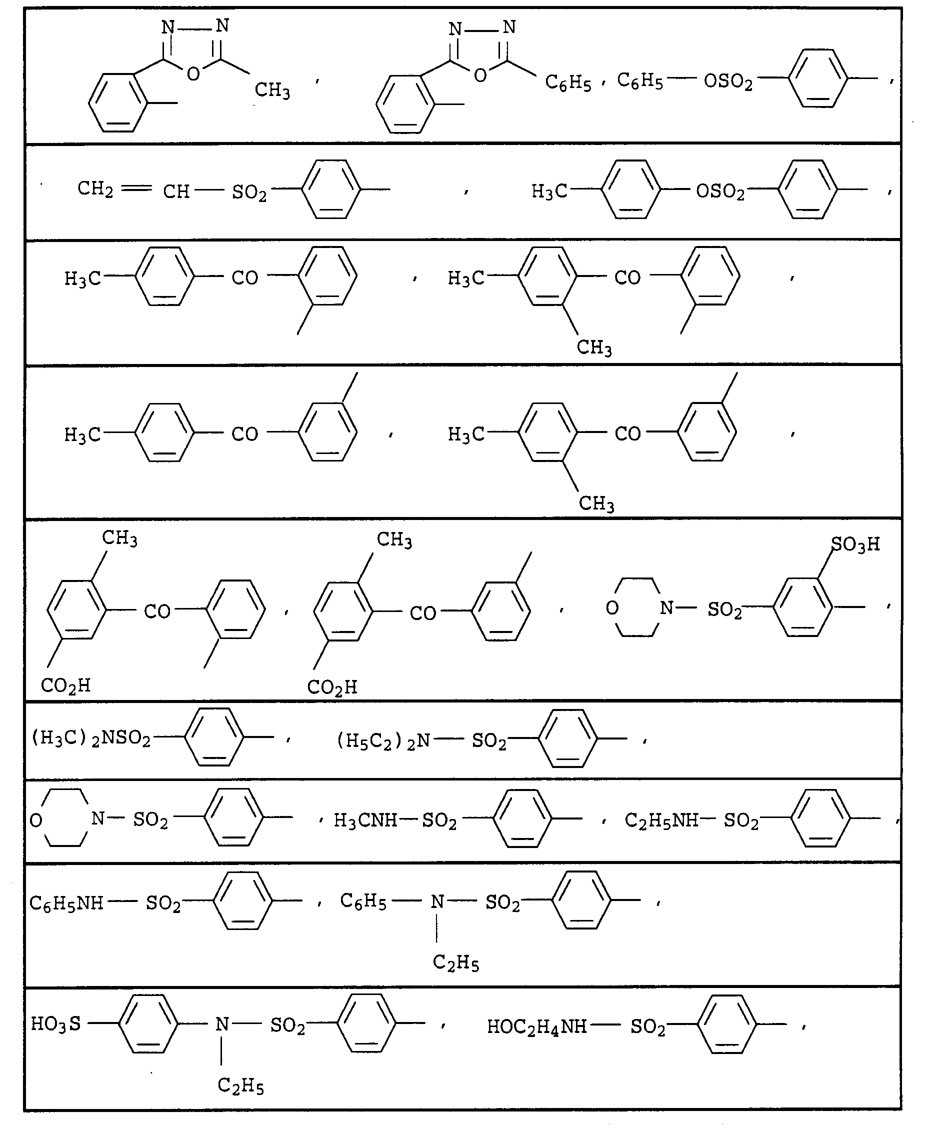

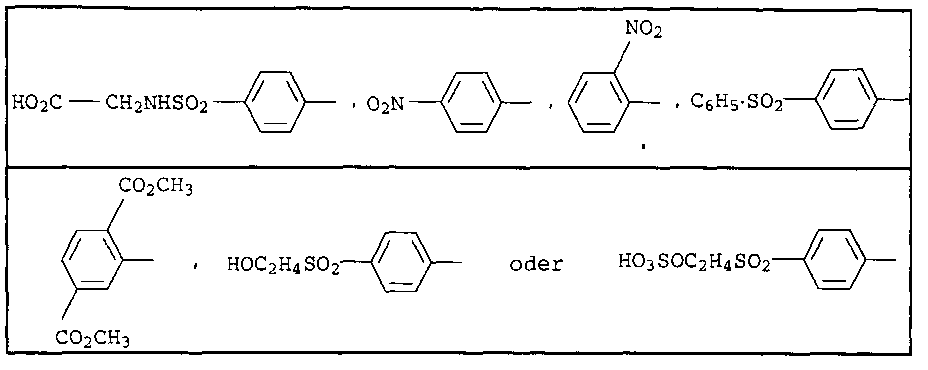

Da die Disazofarbstoffe der Formel I mehrere Hydroxysulfonylgruppen und gegebenenfalls auch Carboxygruppen enthalten, sind auch deren Salze von der Erfindung mit umfaßt.

Als Salze kommen dabei Metall- oder Ammoniumsalze in Betracht. Metallsalze sind insbesondere die Lithium-, Natrium- oder Kaliumsalze. Unter Ammoniumsalzen im erfindungsgemäßen Sinne sind solche Salze zu verstehen, die entweder unsubstituierte oder substituierte Ammoniumkationen aufweisen. Substituierte Ammoniumkationen sind z.B. Monoalkyl-, Dialkyl-, Trialkyl-, Tetraalkyl- oder Benzyltrialkylammoniumkationen oder solche Kationen, die sich von stickstoffhaltigen fünf- oder sechsgliedrigen gesättigten Heterocyclen ableiten, wie Pyrrolidinium-, Piperidinium-, Morpholinium- oder Piperaziniumkationen oder deren N-monoalkyl- oder N,N-dialkylsubstituierte Produkte. Unter Alkyl ist dabei im allgemeinen geradkettiges oder verzweigtes C₁-C₂₀-Alkyl zu verstehen, das durch 1 bis 3 Hydroxygruppen substituiert und/oder durch 1 bis 4 Sauerstoffatome in Etherfunktion unterbrochen sein kann.

Bevorzugt werden Polyazofarbstoffe der Formel I, in der p und q für die Zahl 0 stehen.

Weiterhin bevorzugt sind Polyazofarbstoffe der Formel I, in der Alk für C₁-C₆-Alkyl, das durch 1 oder 2 Sauerstoffatome in Etherfunktion oder 1 Sulfonylgruppe unterbrochen und durch Hydroxy, C₁-C₄-Alkanoyloxy, Sulfato, Chlor, Cyano, Carboxy, Hydroxysulfonyl, Phenyl oder Hydroxysulfonylphenyl substituiert sein kann, steht.

Weiterhin bevorzugt sind Polyazofarbstoffe der Formel I, in der Ar für Phenyl, das durch C₁-C₄-Alkyl, C₁-C₄-Alkoxy, Carboxy, C₁-C₄-Alkanoylamino, Methoxyacetylamino, Hydroxysulfonyl oder einen Rest der Formel SO₂-CH=CH₂, SO₂-C₂H₄-Q, SO₂-NHAlk, SO₂-N(Alk)₂, SO₂-G, SO₂-OG, SO₂-NHG oder SO₂-N(Alk)G substituiert sein kann, steht.

Die Brückenglieder B gehorchen z.B. den Formeln

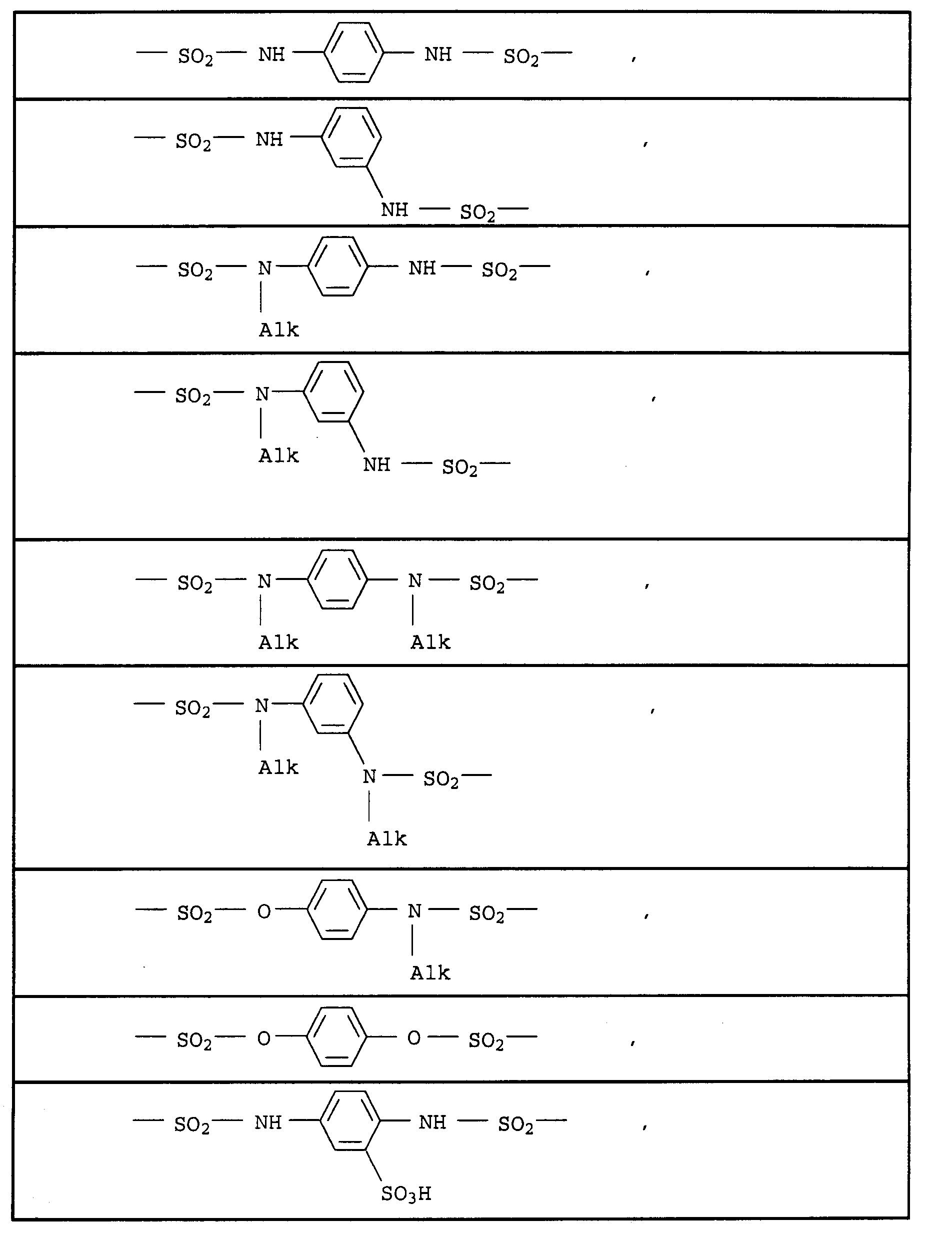

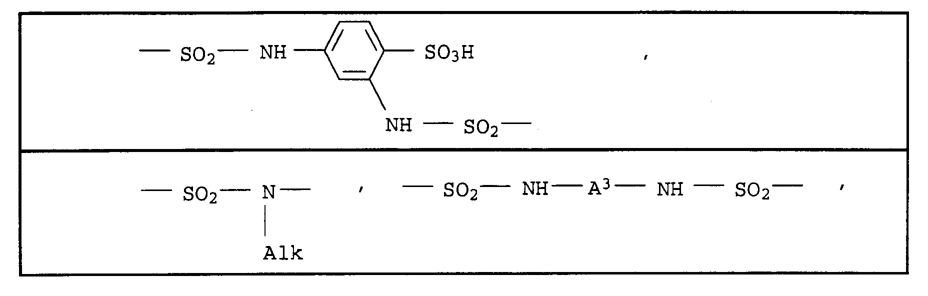

wobei A³ für C₁-C₆-Alkylen steht und Alk die oben angegebene Bedeutung hat. Brückenglieder mit den bevorzugten Resten Alk werden besonders bevorzugt.

Besonders bevorzugt werden als Reste Alk C₁-C₆-Alkyl, das durch 1 oder 2 Sauerstoffatome oder 1 Sulfonylgruppe unterbrochen und durch Hydroxy, C₁-C₄-Alkanoyloxy, Sulfato, Carboxy oder Hydroxysulfonyl substituiert sein kann. In Brückengliedern, die 2 Reste Alk tragen, können die Reste gleich oder verschieden sein z.B.

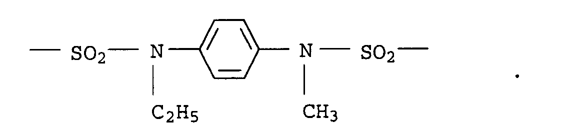

Als Beispiele für die Bisaminoverbindungen von denen sich die Brückenglieder ableiten seien genannt:

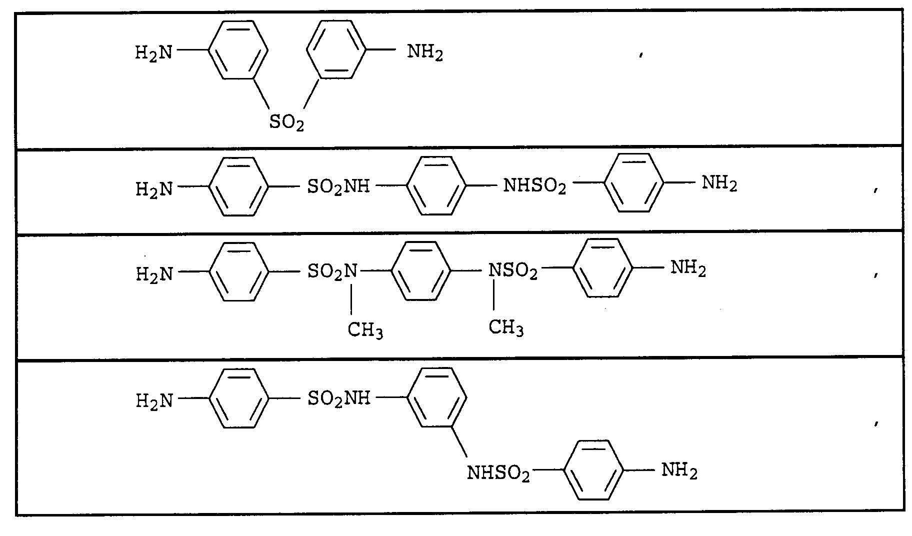

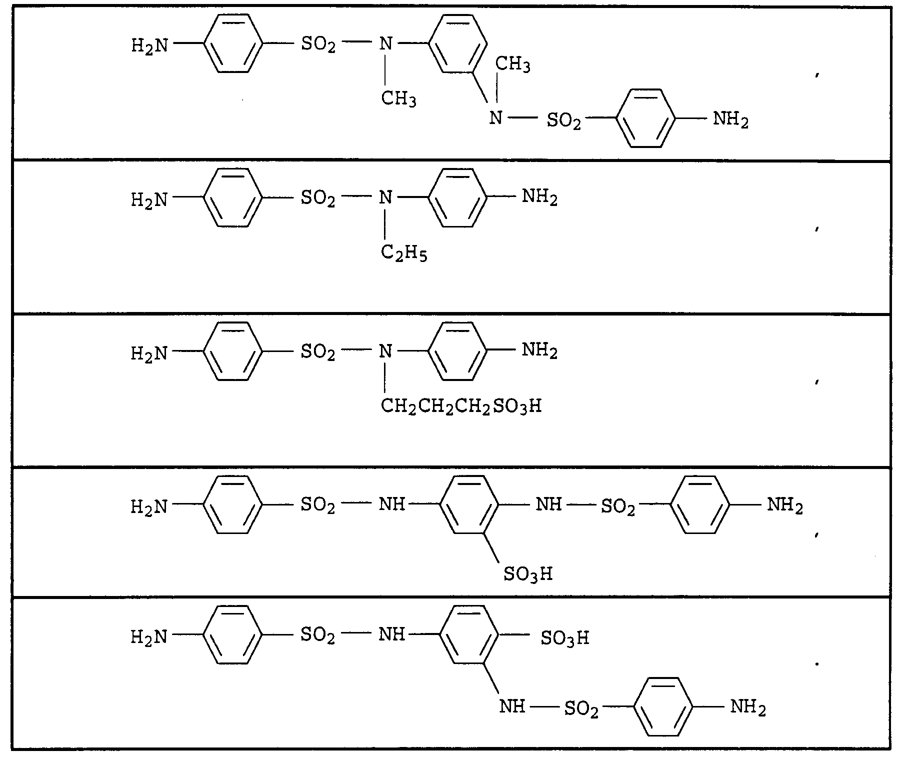

Bevorzugt werden Polyazofarbstoffe mit einem Phenylen(bisaminosulfon)-Brückenglied. Insbesondere die die folgenden Brückenglieder enthaltenden Polyazofarbstoffe werden bevorzugt: -SO₂-NH-A¹-NH-SO₂-, -SO₂-N(Alk)-A¹-NH-SO₂- und -SO₂-N(Alk)-A¹-N(Alk)-SO₂-, wobei Alk und A¹ jeweils die in Anspruch 1 genannte Bedeutung besitzen.

Bevorzugt werden Polyazofarbstoffe der Formel I, in der A¹ für m- oder p-Phenylen steht, das mit Hydroxysulfonyl substituiert sein kann.

Das Brückenglied B ist mit jedem seiner Enden mit den Phenylenringen in m- oder p-Position zur Diazogruppe verknüpft. Bevorzugt werden Polyazofarbstoffe in denen beide Verknüpfungen in p-Position zur Diazogruppe sind.

Polyazofarbstoffe mit mindestens einer N-alkylierten Sulfonamidgruppe im Brückenglied werden besonders bevorzugt.

Insbesondere werden Farbstoffe mit den folgenden 1,4-Phenylenbrückengliedern

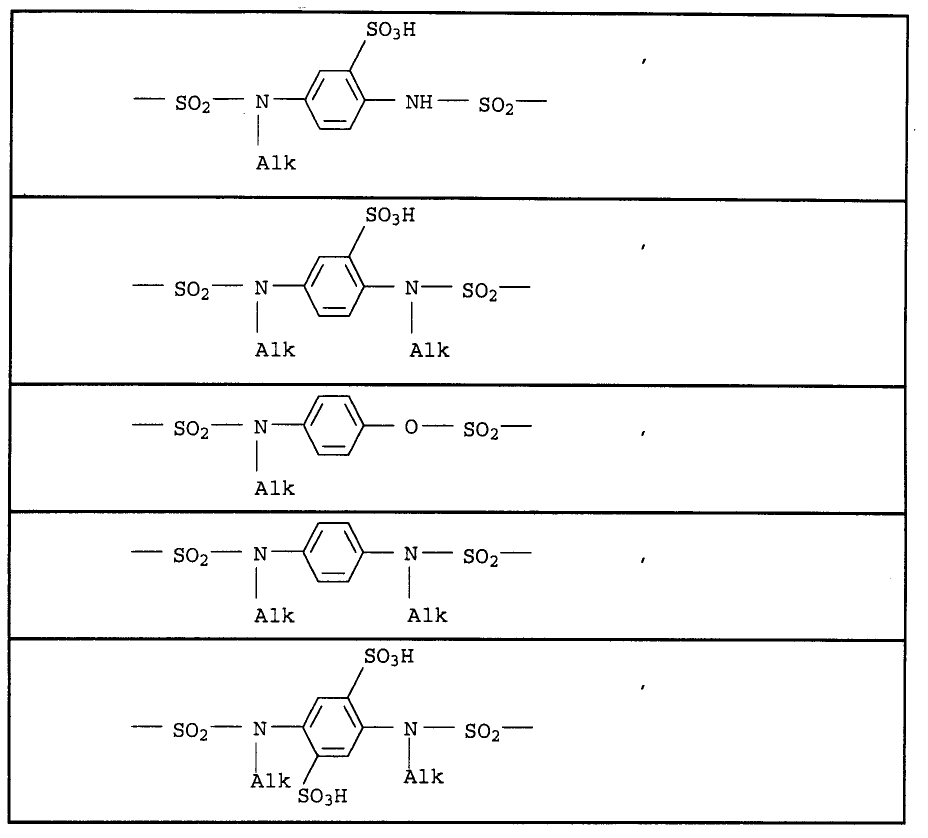

sowie den 1,3-Phenylenbrückengliedern

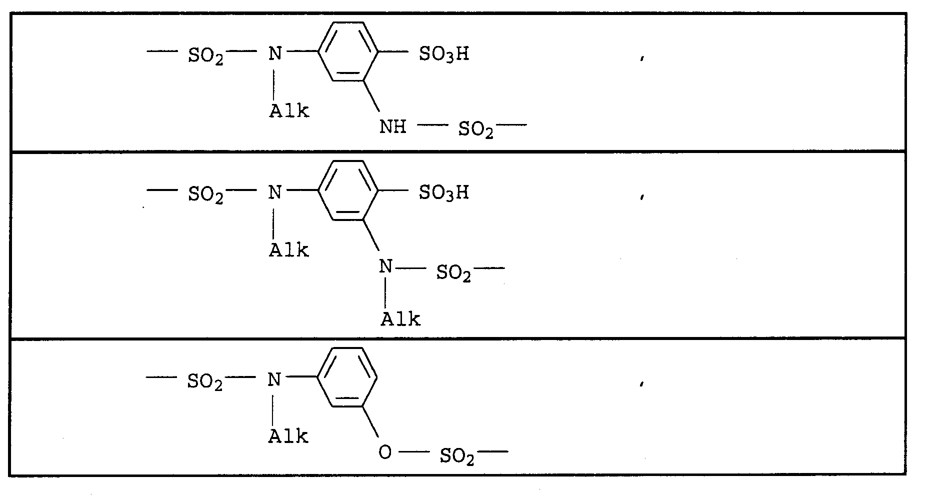

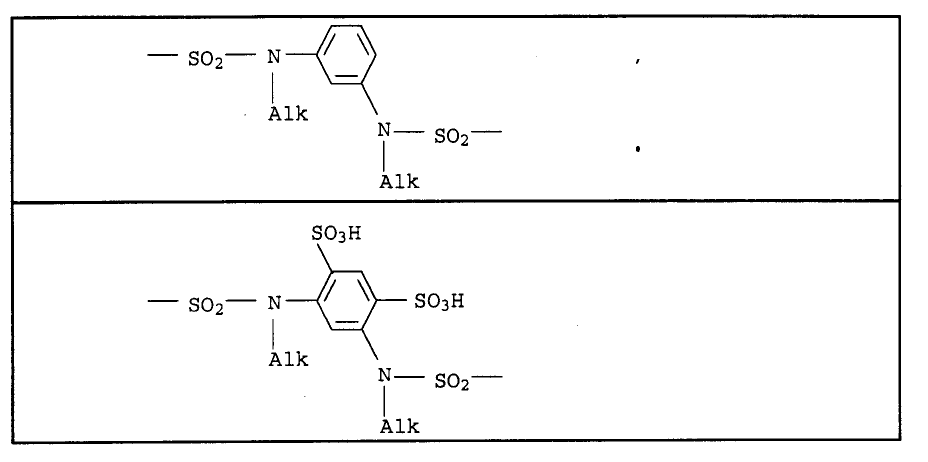

bevorzugt, da ihre Färbungen auf Baumwolle oder Wolle besonders gute Naßechtheiten aufweisen.

Bevorzugt werden Polyazofarbstoffe, in denen von der H-Säure abstammende Farbstoffe verknüpft sind. Insbesondere solche der allgemeinen Formel II 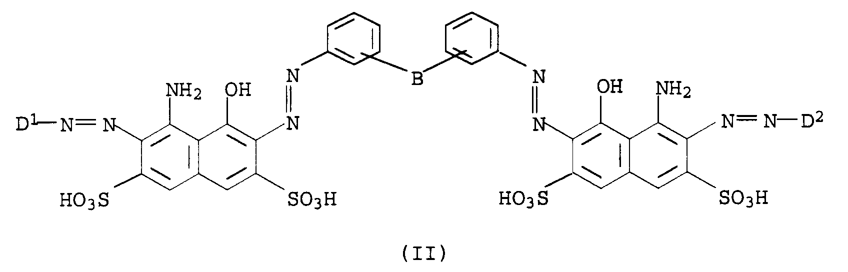 in der D¹, D² und B die oben angegebene Bedeutung haben.

Dabei sind symmetrische Polyazofarbstoffe, also solche, in denen D¹ und D² identisch sind, technisch von besonderem Interesse. Bevorzugt stehen D¹ und D² für einen Rest der Formel 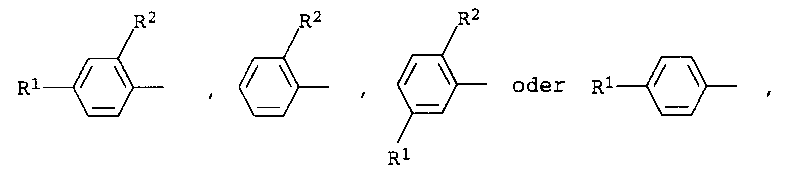 wobei R¹ und R² die oben angegebene Bedeutung haben.

Von ganz besonderer Bedeutung sind Farbstoffe der allgemeinen Formeln IIIa-d. 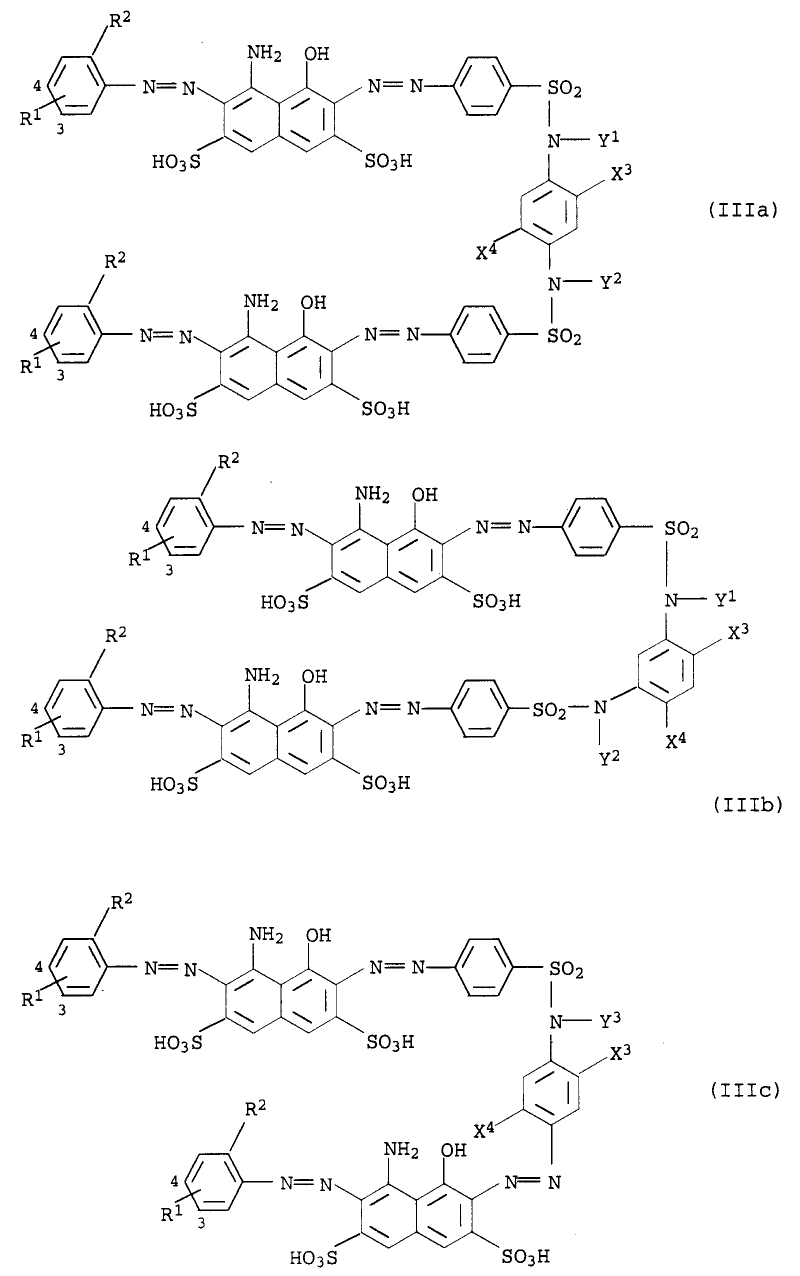 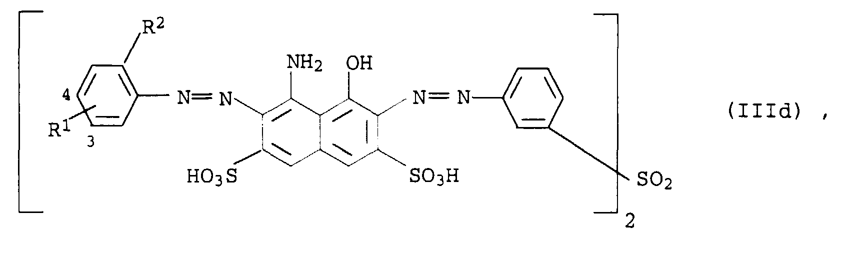 in denen
- R¹: Wasserstoff, Nitro oder ein Rest der Formel HOC₂H₄SO₂, HO₃SOC₂H₄SO₂, CH₂=CH-SO₂, CH₂=CH-CH₂-SO₂, CO-Ar, SO₂-Ar, SO₂-OAr, SO₂-N(Ar)Alk, SO₂-N(Alk)₂, CO-OAlk oder CO-Alk,
- R²: Wasserstoff, C₁-C₄-Alkyl, C₁-C₄-Alkoxy oder einen Rest der Formel CO-OAlk,
- Y¹, Y² und Y³: unabhängig voneinander C₁-C₆-Alkyl, das durch 1 oder 2 Sauerstoffatome oder 1-Sulfonylgruppe unterbrochen und durch Hydroxy, C₁-C₄-Alkanoyloxy, Sulfato, Carboxy oder Hydroxysulfonyl substituiert sein kann oder Y¹ und/oder Y² auch Wasserstoff,
- X³ und X⁴: jeweils Wasserstoff oder Hydroxysulfonyl
bedeuten, wobei der Rest R¹ in 3- oder 4-Position steht und Alk und Ar die oben angegebene Bedeutung haben.

Befindet sich der Rest R¹ in 3-Position, so sind die Reste der Formel CO₂Alk, SO₂C₂H₄OH und SO₂C₂H₄OSO₃H bevorzugte Reste.

Daneben werden Polyazofarbstoffe der Formel I bevorzugt, in denen die Substituenten aus einer Kombination der oben aufgeführten bevorzugten Substituenten ausgewählt sind.

Die neuen Polyazofarbstoffe der Formel I können auf an sich bekannte Weise erhalten werden.

Beispielsweise kann man ein Anilin der Formel IV 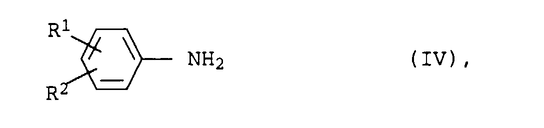 in der R¹ und R² die obengenannte Bedeutung besitzen, auf an sich bekanntem Weg diazotieren und das resultierende Diazoniumsalz mit einem Naphthalin der Formel V 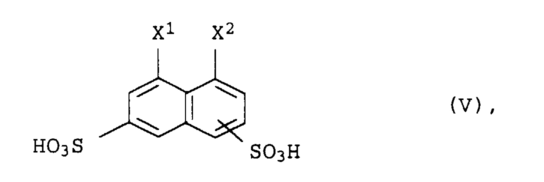 in der X¹ und X² jeweils die obengenannte Bedeutung besitzen, kuppeln.

Der resultierende Monoazofarbstoff der Formel VI 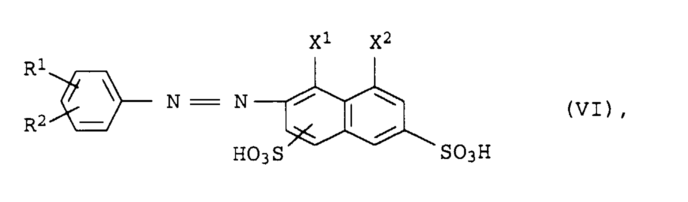 kann dann mit einem Tetraazoniumsalz, das sich von einer Bisaminoverbindung der Formel VII 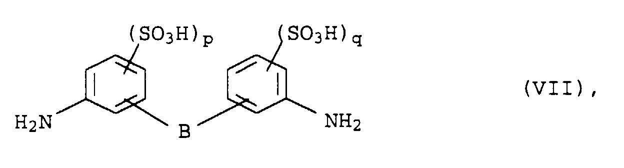 ableitet, worin B, p und q jeweils die obengenannte Bedeutung besitzen, gekuppelt werden.

Die erfindungsgemäßen Farbstoffe der Formel I eignen sich in vorteilhafter Weise zum Färben von natürlichen oder synthetischen Substraten, beispielsweise von Baumwolle, Wolle, Leder oder Polyamid.

Man erhält Färbungen in schwarzen sowie blauen bis marineblauen Farbtönen mit guter Lichtechtheit sowie guten Naßechtheiten.

Die neuen Farbstoffe können sowohl alleine wie auch in Mischung untereinander oder auch in Mischung mit anderen Farbstoffen zur Anwendung gelangen.

Ein weiterer Gegenstand der vorliegenden Erfindung sind Sulfonamide der Formel (VIIa) 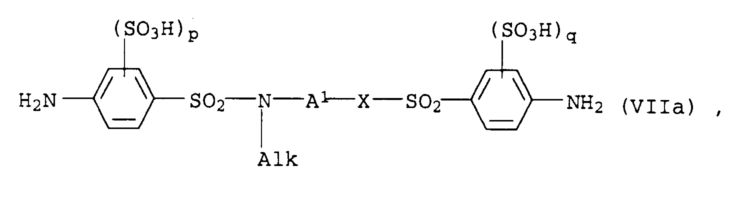 in der
- p: die Zahl 0 oder 1,
- q: die Zahl 0 oder 1,
- A¹: Phenylen, das mit Hydroxysulfonyl substituiert sein kann,
- X: Sauerstoff, Imino oder einen Rest 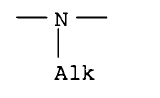 und
- Alk: C₁-C₈-Alkyl, das durch 1 bis 3 Sauerstoffatome in Etherfunktion oder durch 1 Schwefelatom oder 1 Sulfonylgruppe unterbrochen und durch Hydroxy, C₁-C₄-Alkanoyloxy, Benzoyloxy, Sulfato, Halogen, Cyano, Carbamoyl, Carboxy, Hydroxysulfonyl, Phenyl oder Hydroxysulfonylphenyl substituiert sein kann, oder C₅-C₈-Cycloalkyl
bedeuten.

Ein weiterer Gegenstand der vorliegenden Erfindung sind Sulfonamide der Formel VIIb 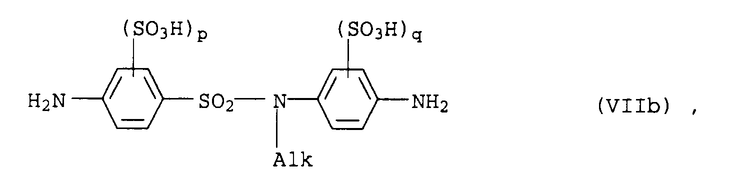 in der
- p: die Zahl 0 oder 1,
- q: die Zahl 0 oder 1 und
- Alk: C₁-C₈-Alkyl, das durch 1 bis 3 Sauerstoffatome in Etherfunktion oder durch 1 Schwefelatom oder 1 Sulfonylgruppe unterbrochen und durch Hydroxy, C₁-C₄-Alkanoyloxy, Benzoyloxy, Sulfato, Halogen, Cyano, Carbamoyl, Carboxy, Hydroxysulfonyl, Phenyl oder Hydroxysulfonylphenyl substituiert sein kann, oder C₅-C₈-Cycloalkyl
bedeuten.

Bezüglich der beispielhaften Aufzählung der Substituenten Alk sei auf die oben gemachten Ausführungen verwiesen.

Die neuen Sulfonamide der Formel VIIa können auf an sich bekanntem Wege erhalten werden.

Beispielsweise kann man ein Anilin der Formel VIII 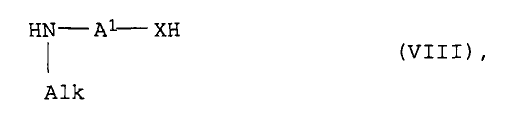 in der Alk, A¹ und X jeweils die obengenannte Bedeutung besitzen, zweifach mit 4-Acetylaminobenzolsulfonsäurechlorid zum Sulfonamid umsetzen und anschließend entacetylieren.

Die Sulfonamide der Formel VIIb sind entsprechend durch einfache Umsetzung eines Nitroaminobenzols der Formel IX 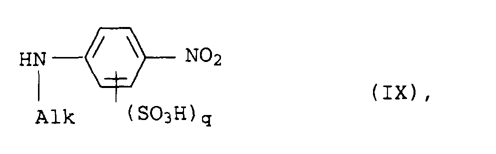 mit 4-Acetylaminobenzolsulfonsäurechlorid und anschließender Hydrierung und Entacetylierung erhältlich.

Ein weiterer Zugang zu den Verbindungen der Formel VIIa und VIIb ist die Alkylierung der Iminogruppe der aminogeschützten, z.B. durch Acetylgruppen, Verbindungen der Formel X 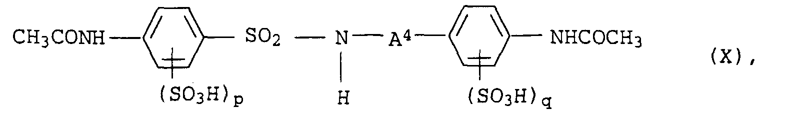 worin A⁴ für eine direkte Bindung oder ein Brückenglied der Formel -A¹-X-SO₂- steht, dessen Sulfonylgruppe mit dem Benzolring verknüpft ist. Die Alkylierung läßt sich, wie allgemein bekannt, in polaren Lösungsmitteln wie Wasser, Aceton oder Alkoholen in alkalischen pH-Bereich von 7 bis 12 mit Alkylierungsmitteln wie Dimethylsulfat, Diethylsulfat oder Propansulton durchführen. Die Entschützung führt zu den Verbindungen der Formeln VIIa und VIIb.

Die hier nicht explizit aufgeführten Zwischenprodukte sind wohlbekannt und können auf dem Fachmann wohlbekannten Wege erhalten werden.

Die folgenden Beispiele sollen die Erfindung näher erläutern.

### Beispiel 1

a) 281 g der Diazokomponente der Formel 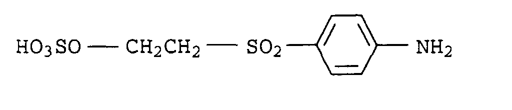 wurden mit ca. 85 g Natriumhydrogencarbonat in 1000 ml Wasser bei pH 3,5 gelöst und durch anschließende Zugabe von 227 ml 36 gew.-%iger Salzsäure gefällt. Die Mischung wurde mit Eis auf 0°C abgekühlt und bei Temperaturen unter 5°C durch Zutropfen von 305 ml wäßriger 23 gew.-%iger Natriumnitritlösung diazotiert. Nach Zerstören von überschüssiger salpetriger Säure mit Amidosulfonsäure gab man die Diazoniumsalzsuspension zu einer salzsauren Fällung von 319 g 1-Amino-8-hydroxynaphthalin-3,6-disulfonsäure, welche zuvor in 2200 ml Natronlauge gelöst worden war. Die Suspension wurde 12 Stunden bei Raumtemperatur gerührt.
b) 108 g 3,3' -Diaminodiphenylsulfon wurden bei Raumtemperatur eine Stunde in 600 ml 18,5 gew.-%iger Salzsäure in 150 ml Wasser gerührt. Die Mischung wurde auf Temperaturen unter 0°C gekühlt und über einen Zeitraum von 30 bis 45 min 310 ml einer 23 gew.-%igen Natriumnitritlösung zudosiert. Es wurde eine Stunde nachgerührt. Die überschüssige salpetrige Säure wurde zerstört und der pH-Wert der Lösung mit Natriumhydrogencarbonat auf 2,0 gestellt. Die so hergestellte Tetrazoniumsalzlösung wurde innerhalb von 30 min zu der unter a) hergestellten Farbstoff lösung gegeben, die zuvor mit Natriumcarbonat auf pH 7,0 gestellt worden war. Man erhielt einen blauen Farbstoff der Formel 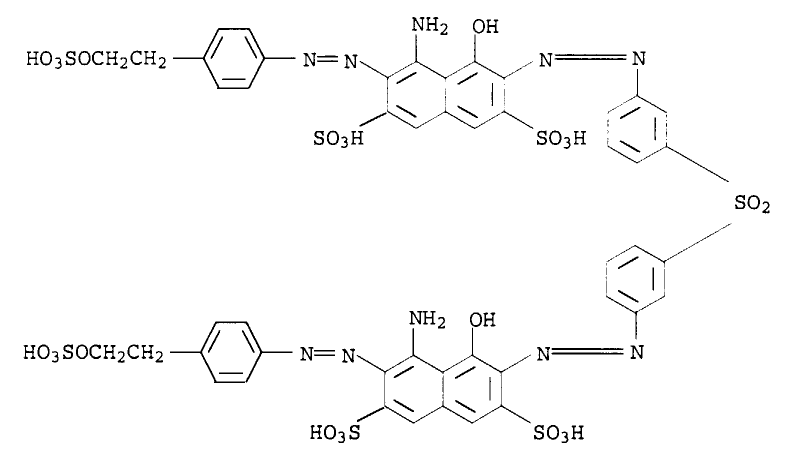 in Form seiner freien Säure, die mit Natriumhydrogencarbonat gleichzeitig gebunden wurde.
   Anschließend stellt man den pH-Wert der Kupplung mit wenig Natriumphosphat auf 6,0 - 6,3 und engte dann die Kupplungslösung durch Sprühtrocknung ein. Man erhielt 1650 g eines blauschwarzen Pulvers, das Baumwolle im Ausziehverfahren bei 40 - 60°C in kräftigem Marineblau mit hohen Naßechtheiten färbte. λₘₐₓ der ca. 0,01 %igen wäßrigen Lösung vom pH ca. 4: 586 nm.

### Beispiel 2

Setzte man anstelle von 3,3-Diaminodiphenylsulfon 223 g der Verbindung der Formel 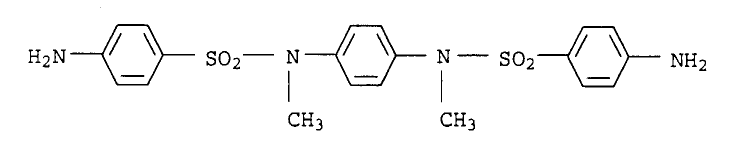 analog zu Beispiel 1 ein, so erhielt man nach Sprühtrocknung den Farbstoff der Formel 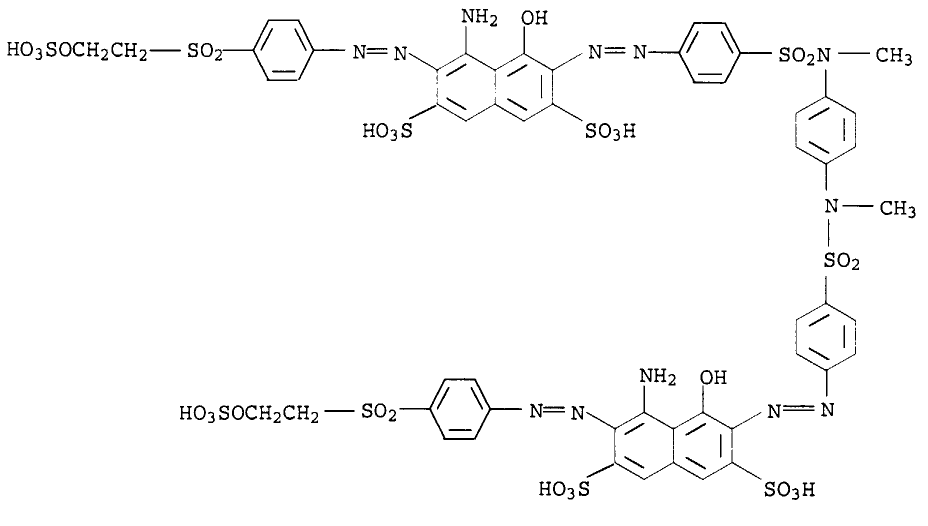 in Form eines schwarzen Pulvers.

Die Lösung des Farbstoffes in Wasser vom pH ca. 4 hat ein Absorptionsmaximum bei 583 nm.

Der Farbstoff färbt Baumwolle in farbstarken und naßechten marineblauen Nuancen an.

### Beispiel 3

209 g der Tetrazokomponente der Formel 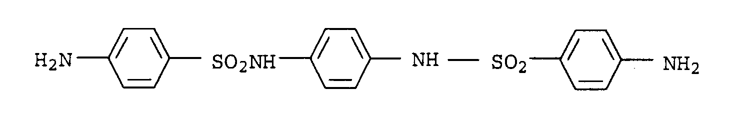 wurden 12 h mit 900 ml 18,5 gew.-%iger Salzsäure verrührt. Dann kühlte man das Gemisch mit Eis auf < 0°C ab und tropfte innerhalb von 10 min 305 ml einer 23 gew.-%igen, wäßrigen Natriumnitritlösung hinzu. Die entstehende sandfarbene Suspension wurde mit 500 ml 5°C kaltem Wasser verdünnt und 1 h bei ca. 5°C nachgerührt. Dann zerstörte man überschüssige salpetrige Säure und dosierte die Suspension zu 468 g der Kupplungskomponente der Formel 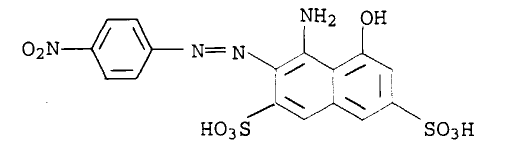 welche zuvor in 2000 ml Wasser bei pH 7-8 gelöst wurde. Gleichzeitig hielt man den pH-Wert der Kupplung mit Natriumcarbonat im Bereich von 6,5 bis 9. Anschließend wurde die Mischung 40 min bei pH 8,5 gerührt, dann mit Salzsäure auf pH 4,5 angesäuert und bei 80°C mit Kochsalz versetzt.

Der Farbstoff der Formel 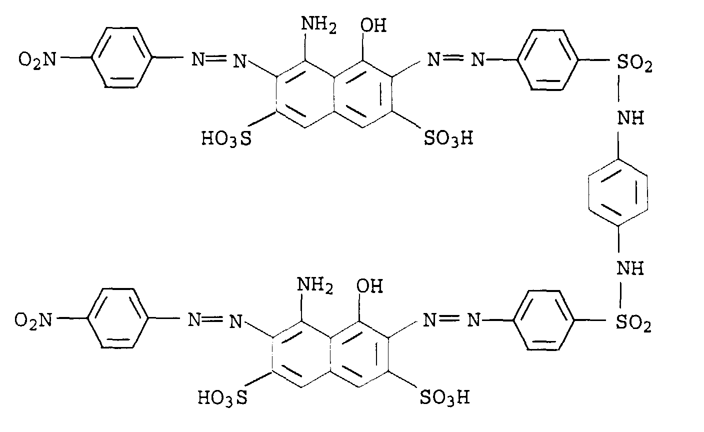 fiel (als Natriumsalz) in gut filtrierbarer Form an. Nach Absaugen und Trocknen erhielt man 1220 g eines schwarzen Pulvers das sich in Wasser mit rotstichig blauem Farbton löste. λₘₐₓ(pH=4): 560 nm (Schulter bei ca. 620 nm).

Der Farbstoff färbte Polycaprolactamgewebe in blauen bis tiefen marineblauen Tönen mit vorzüglichen Naßechtheiten.

### Beispiel 4

Man verfuhr analog zu Beispiel 3, verwendete aber 563 g der Kupplungskomponente der Formel 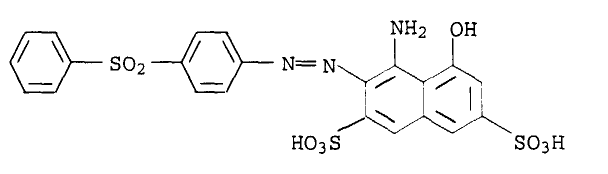 (hergestellt durch saure Kupplung des Diazoniumsalzes Diphenylsulfon-4-diazoniumchlorid auf 1-Amino-8-hydroxynaphthalin-3,6-disulfonsäure bei pH < 2).

Nach Isolieren des Farbstoffes analog zu Beispiel 3 erhielt man ein schwarzes Pulver der Formel

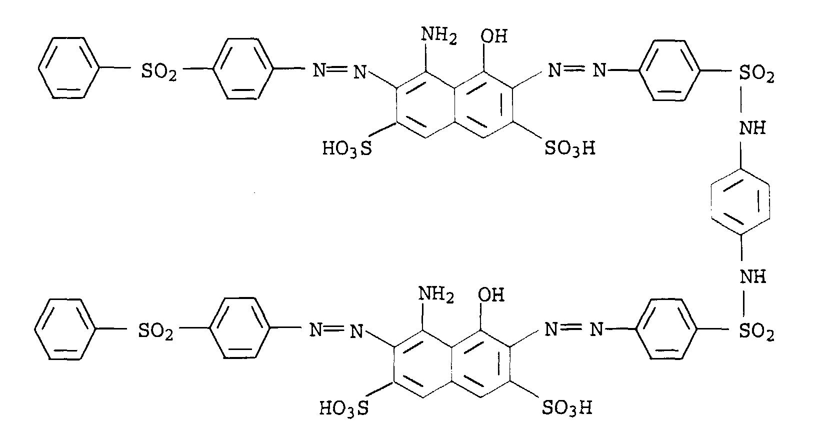

Der Farbstoff löste sich in Wasser mit rotstichig, blauem Farbton und färbte Polycaprolactamgewebe in echtem marineblauem Farbton an.

Analog Beispiel 2 wurden die folgenden Farbstoffe erhalten:

### Beispiel 5

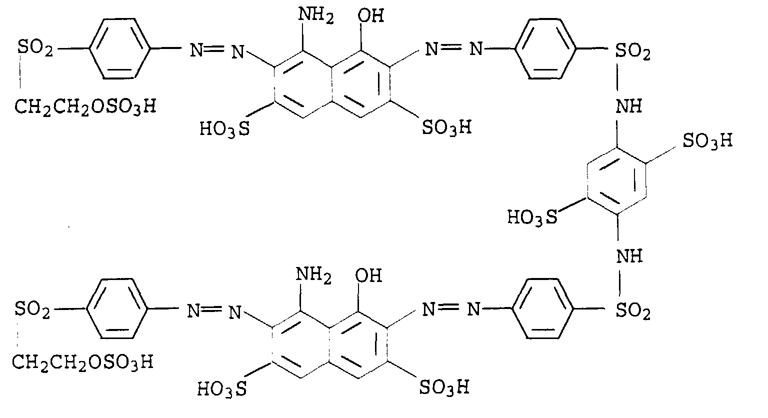

Der Farbstoff färbt Baumwolle in marineblauen Tönen.

### Beispiel 6

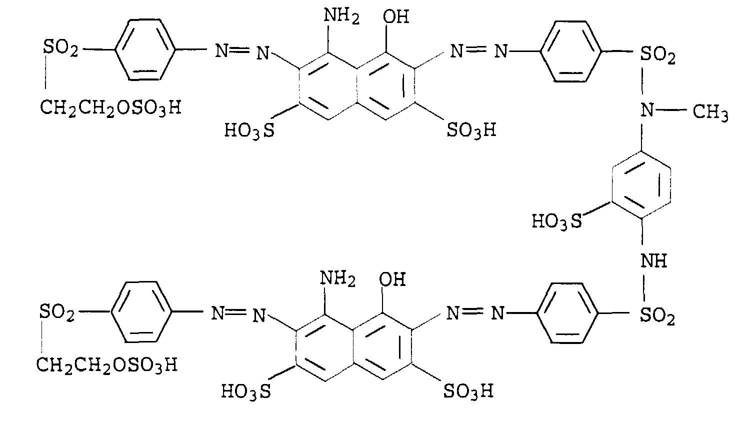

Der Farbstoff färbt Baumwolle in marineblauen Tönen.

### Beispiel 7

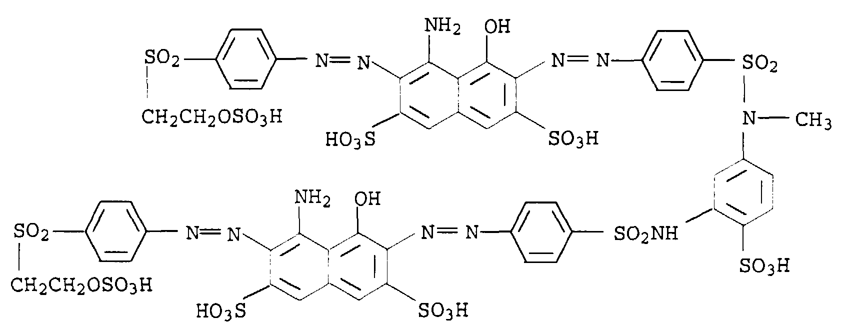

Der Farbstoff färbt Baumwolle in marineblauen Tönen.

### Beispiel 8

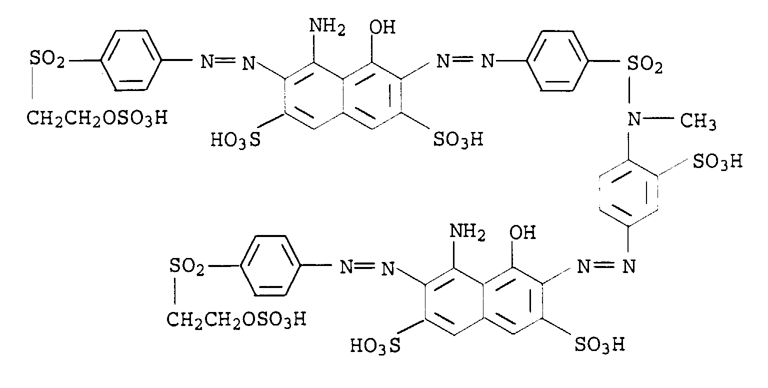

Der Farbstoff färbt Baumwolle in marineblau-schwarzen Tönen.

### Beispiel 9

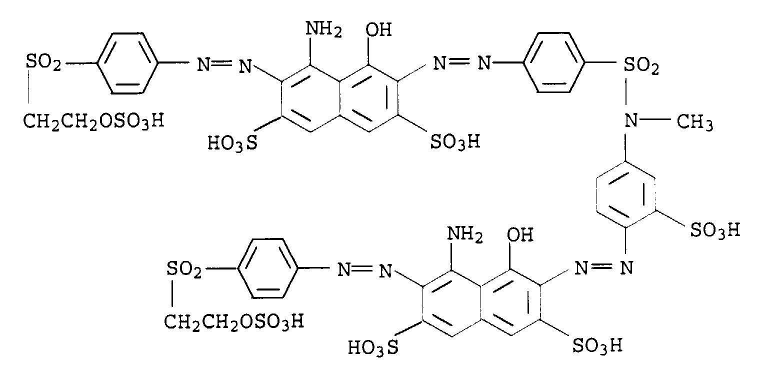

Der Farbstoff färbt Baumwolle in marineblau-schwarzen Tönen.

### Beispiel 10

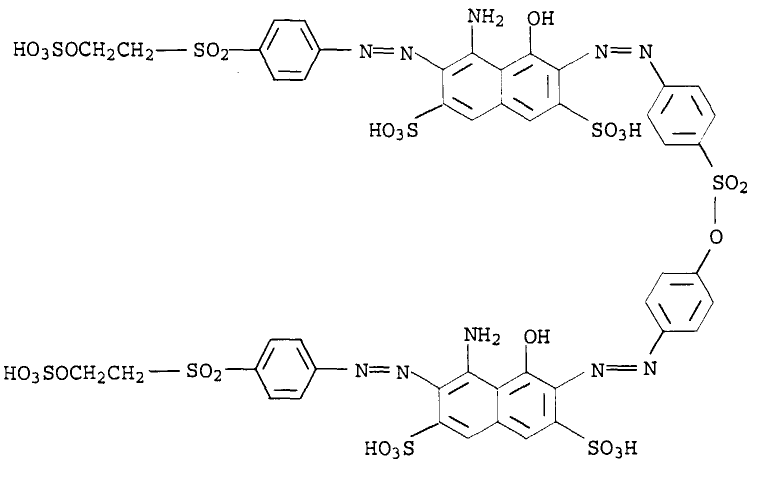

Der Farbstoff färbt Baumwolle in blaustichig-schwarzen Tönen (λₘₐₓ = 600 nm breit).

Analog zu den Beispielen 2, 3 und 4 wurden die in den folgenden Tabellen genannten Farbstoffe hergestellt.

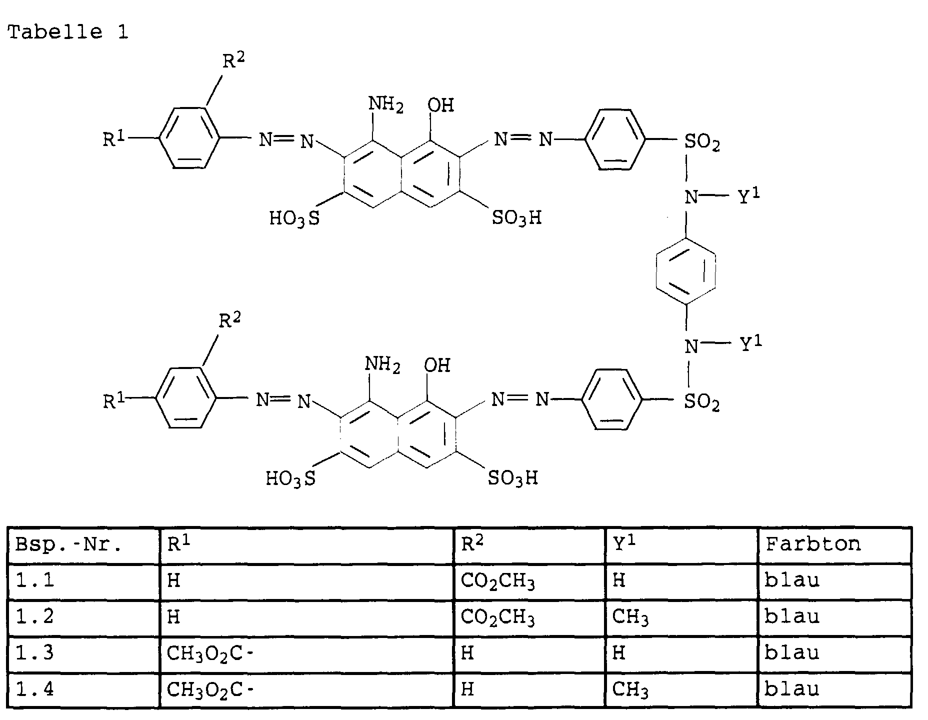

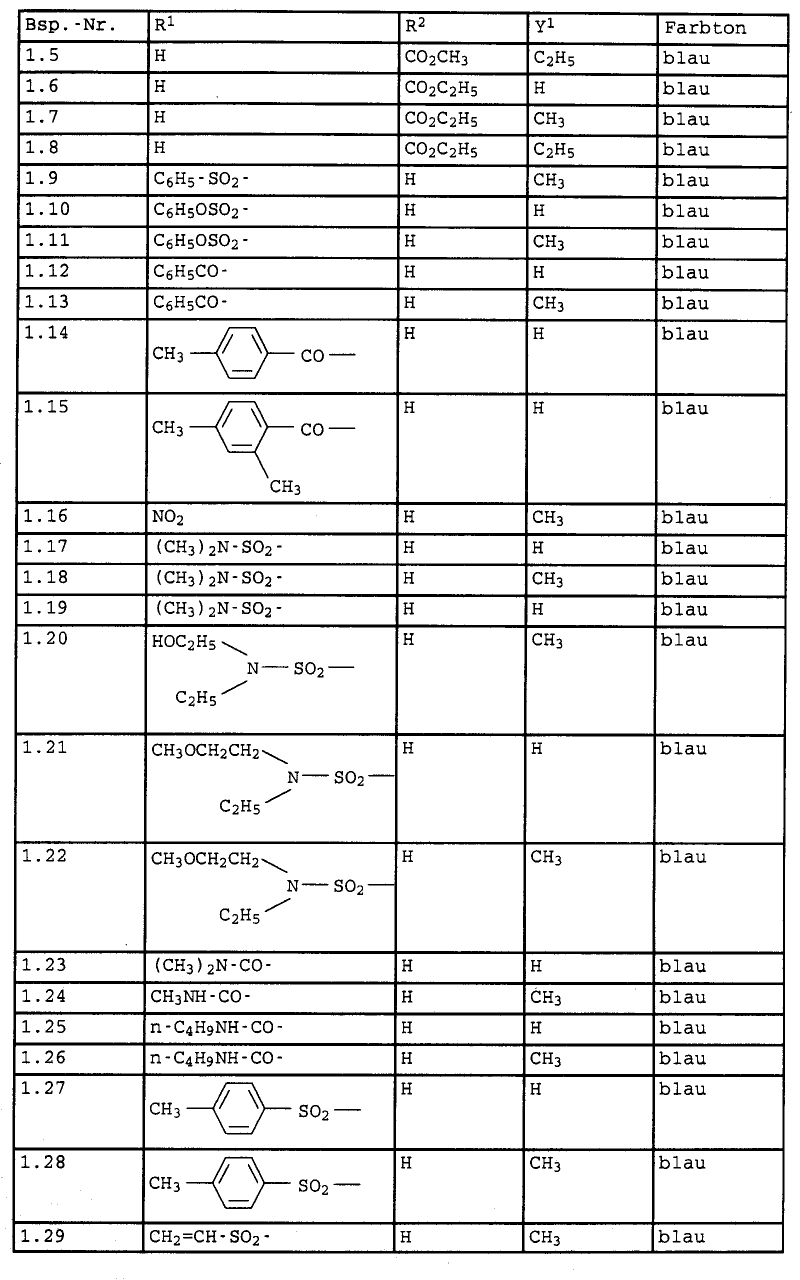

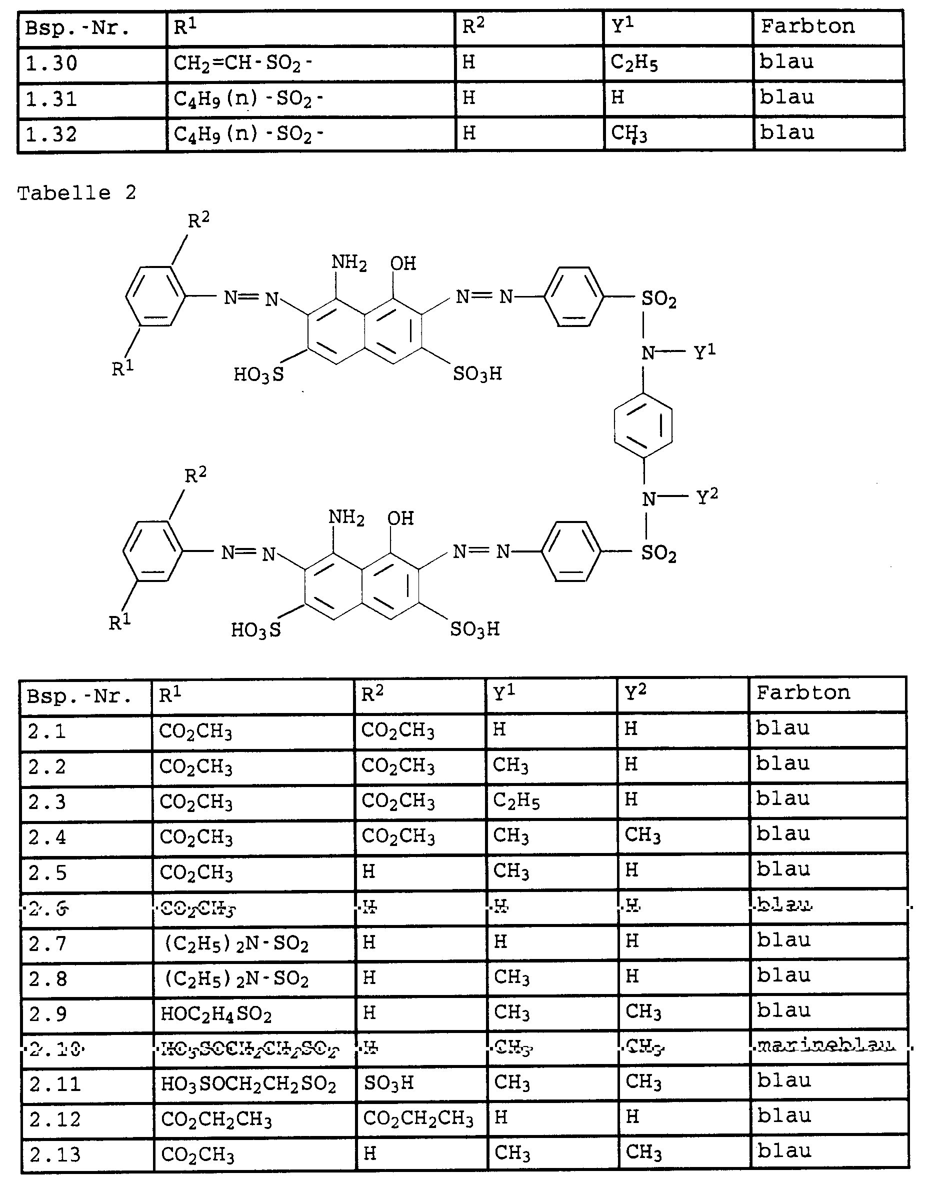

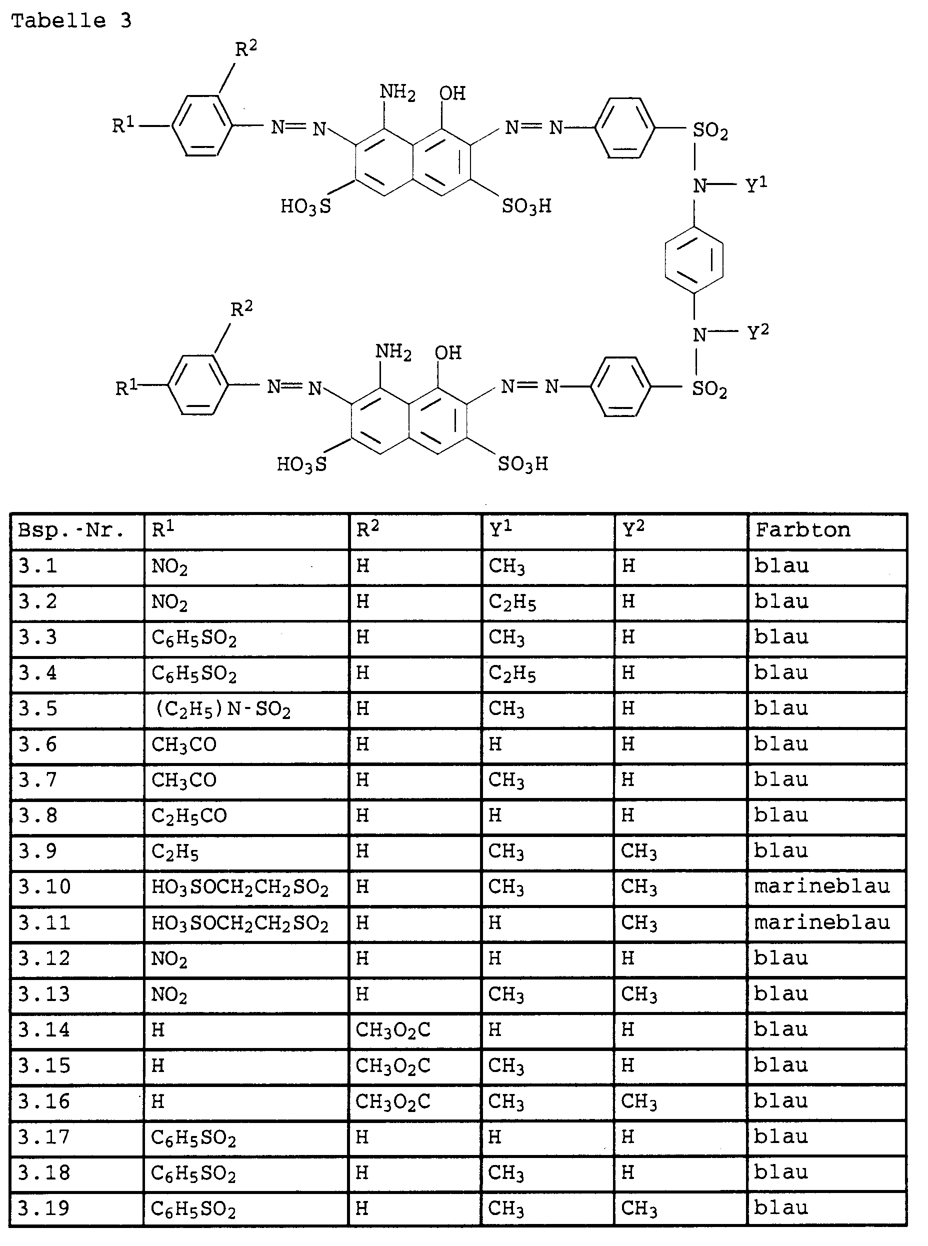

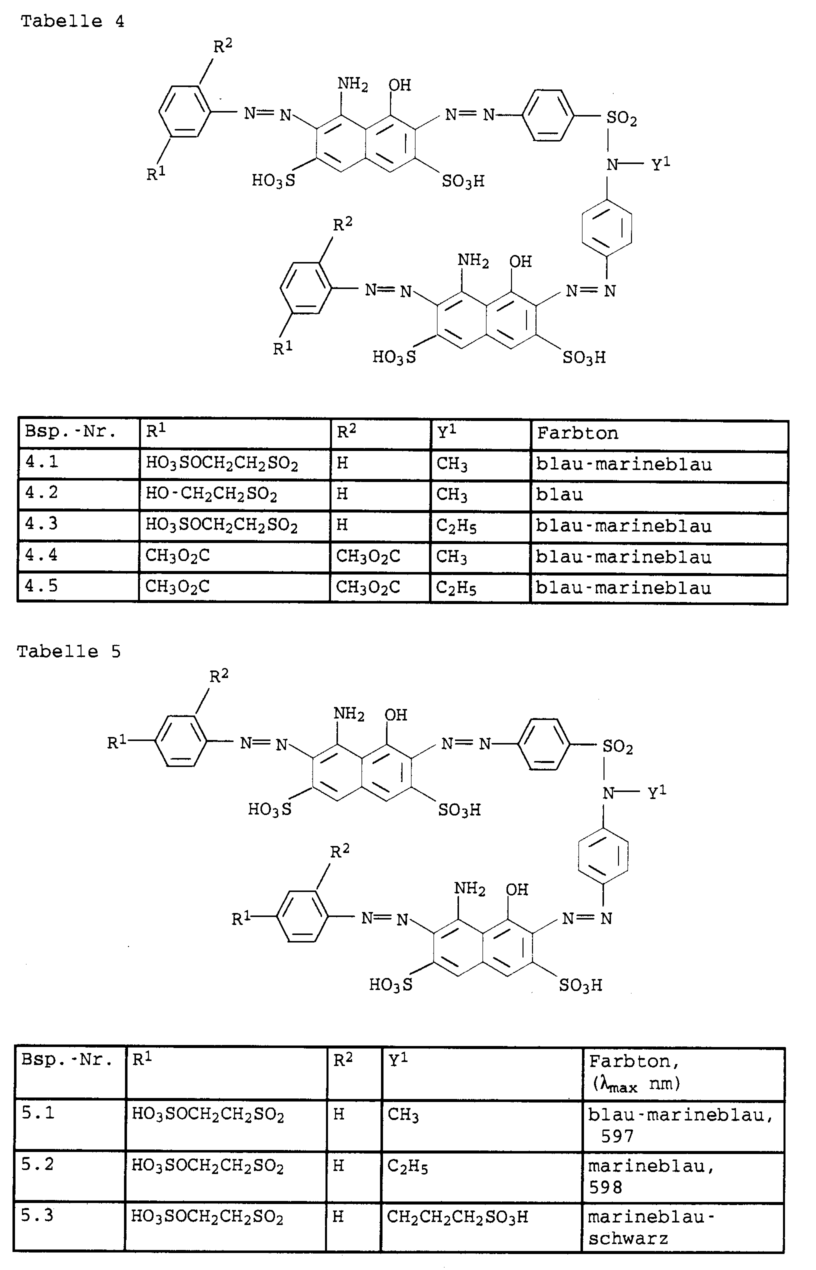

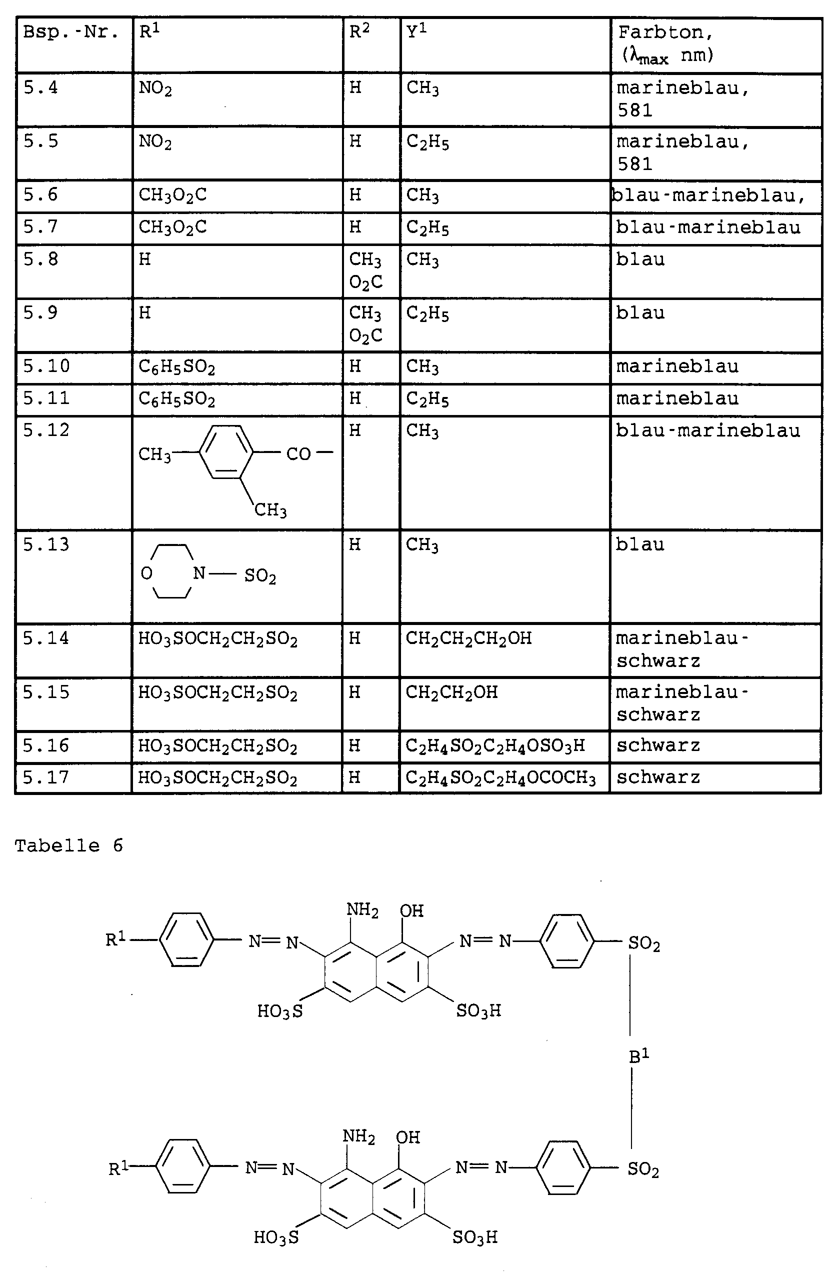

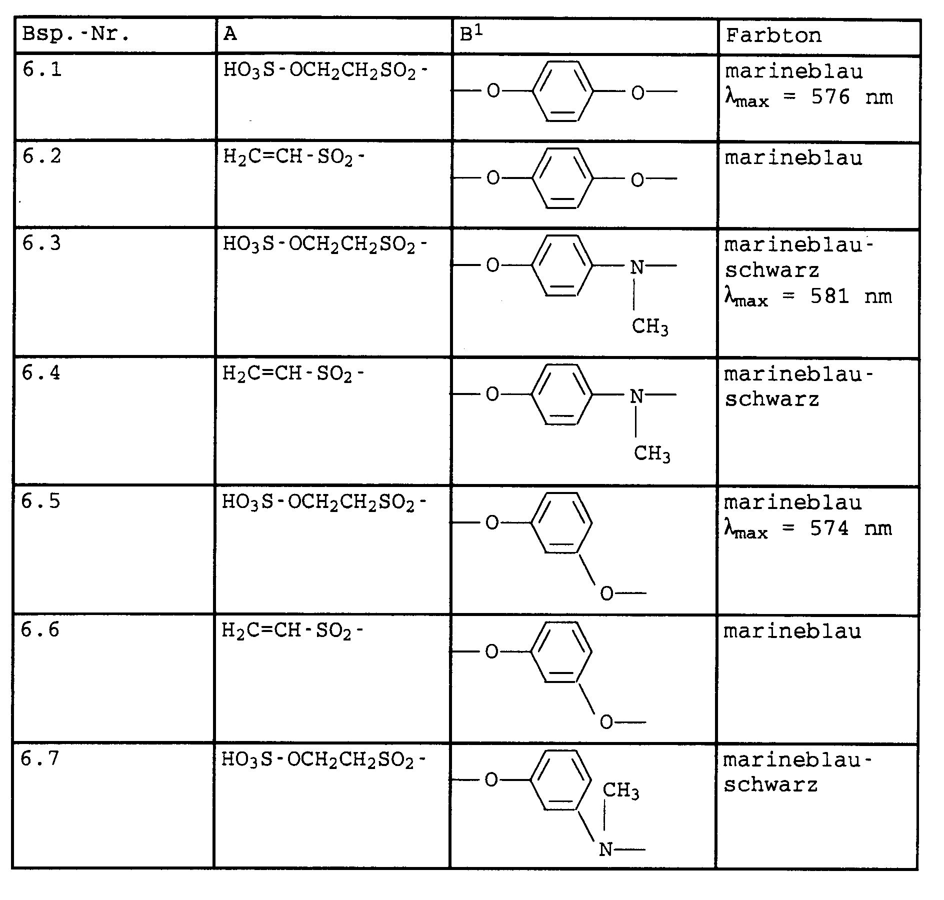

### Beispiel 11

188 g 1,4-Diaminobenzol-2-sulfonsäure (ber. 100 %) wurden in 1900 ml Wasser mit Natronlauge bei pH 7 gelöst. Man heizte auf 50°C auf und dosierte unter kräftigem Rühren innerhalb von 1 Std. portionsweise 450 g gemahlenes p-Acetaminobenzolsulfonylchlorid (ber. 100 %) zu und gab gleichzeitig zunächst 25 g Soda und danach 25 gew.-%ige Natronlauge so hinzu, daß der pH-Wert des Gemisches anfangs 7-8 und später, nachdem ca. 50 % des Sulfochlorids eingetragen wurden, 7,5 bis 8,5 betrug. Anschließend rührte man die erhaltene Suspension noch 1 Std. bei 50°C nach. Dann wurde der pH-Wert des Gemisches mit Natronlauge auf 10,5 gestellt. Danach tropfte man innerhalb von 30 Min. 138 g Dimethylsulfat hinzu und hielt den pH-Wert des Gemisches dabei mit Natronlauge bei 10-11. Man rührte 30 Min. bei 40-50°C nach, gab 20 g 25 gew.-%iger wäßriger Ammoniaklösung hinzu, stellte den pH-Wert des Gemisches mit 50 gew.-%iger Natronlauge auf 14 und deacetylierte innerhalb von 7 Std. bei 90-95°C bei einem pH-Wert von 13 bis 14. Dazu wurden insgesamt ca. 170 g 50 gew.-%ige Natronlauge nachgesetzt. Dann ließ man erkalten und neutralisierte mit 18,5 gew.-%iger Salzsäure. Das ausgefallene farblose Produkt der Formel 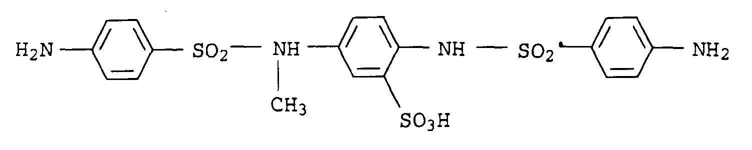 wurde bei pH 6-7 abgesaugt und mit kaltem Wasser gewaschen. Man erhielt 480 g eines farblosen Pulvers.

### Beispiel 12

Man verfuhr analog zu Beispiel 11, setzte aber anstelle der 1,4-Diaminobenzol-2-sulfonsäure 244 g der Verbindung der Formel 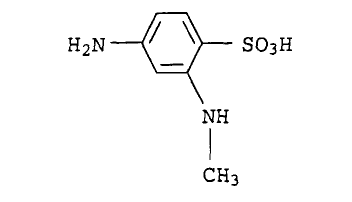 ein. Nach Deacetylierung bei pH > 12 und anschließender Neutralisation erhielt man analog zu Beispiel 11 500 g der Tetraazokomponente der Formel

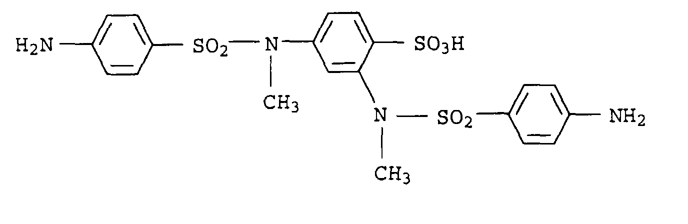

## Patentansprüche

1. Polyazofarbstoffe der Formel I 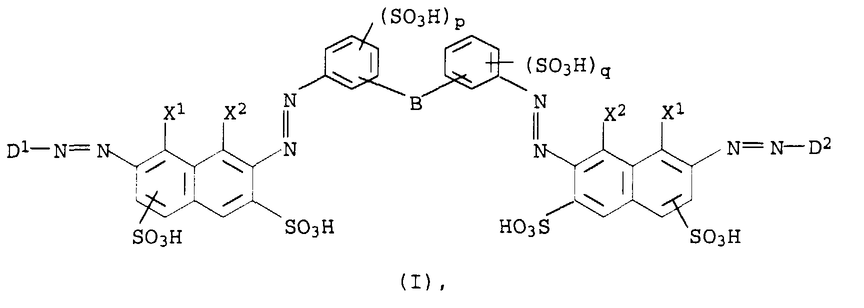 in der einer der beiden Reste X¹ und X² Hydroxy und der andere Amino,
p die Zahl 0 oder 1,
q die Zahl 0 oder 1,
D¹ und D² unabhängig voneinander einen Rest der Formel 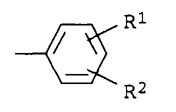
B ein Brückenglied der Formel
-SO₂-NH-A¹-NH-SO₂-, -SO₂-N(Alk)-A¹-NH-SO₂-, -SO₂-N(Alk)-A¹-N(Alk)-SO₂-, -SO₂-N(Alk)-A¹-O-SO₂-, -SO₂-O-A¹-O-SO₂-, -SO₂-N(Alk)-, -SO₂-N(Alk)-SO₂-, -SO₂-NH-A²-NH-SO₂-, -SO₃- oder SO₂ bedeuten, wobei
A¹ für Phenylen, das mit Hydroxysulfonyl substituiert sein kann
A² für C₁-C₈-Alkylen
R¹ für Wasserstoff, C₁-C₄-Alkyl, Halogen, Cyano, Nitro, Hydroxysulfonyl, Pyrrolidinylsulfonyl, Piperidinylsulfonyl, Morpholinylsulfonyl oder einen Rest der Formel CO-Ar, CO-OAr, CO-Alk, CO-OAlk, CO-N(Ar)Alk, CO-N(Alk)₂, SO₂-Ar, SO₂-Alk, SO₂-CH₂CH=CH₂, SO₂-CH=CH₂, SO₂-C₂H₄-Q, SO₂-OAr, SO₂-N(Alk)₂, SO₂-NHAlk, SO₂-N(Ar)Alk, SO₂-NHAr, 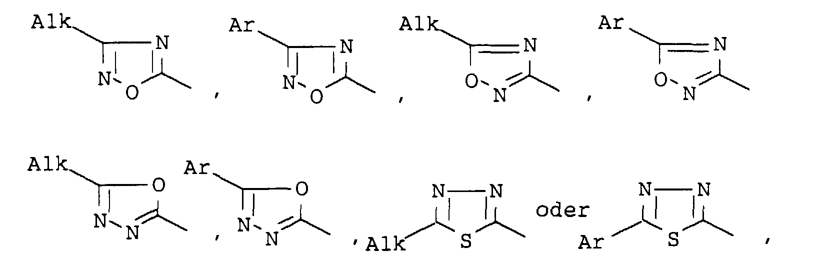
Alk für C₁-C₈-Alkyl, das durch 1 bis 3 Sauerstoffatome in Etherfunktion oder durch 1 Schwefelatom oder 1 Sulfonylgruppe unterbrochen sein kann und durch Hydroxy, C₁-C₄-Alkanoyloxy, Benzoyloxy, Sulfato, Halogen, Cyano, Carbamoyl, Carboxy, Hydroxysulfonyl, Phenyl oder Hydroxysulfonylphenyl substituiert sein kann, oder C₅-C₈-Cycloalkyl,
Ar für Phenyl oder Naphthyl, wobei diese Reste jeweils durch C₁-C₄-Alkyl, Halogen, C₁-C₄-Alkoxy, Phenoxy, Hydroxy, Carboxy, C₁-C₄-Alkanoylamino, dessen Alkylkette durch 1 Sauerstoffatom in Etherfunktion unterbrochen sein kann, Benzoylamino, Hydroxysulfonyl, Pyrrolidinylsulfonyl, Piperidinylsulfonyl, Morpholinylsulfonyl oder einen Rest der Formel SO₂-Alk, SO₂-CH₂CH=CH₂, SO₂-CH=CH₂, SO₂-C₂H₄-Q, SO₂-NHAlk, SO₂-N(Alk)₂, SO₂-G, SO₂-OG, SO₂-NHG oder SO₂-N(Alk)G substituiert sein können,
R² für Wasserstoff, Hydroxysulfonyl, C₁-C₄-Alkyl, C₁-C₄-Alkoxy oder einen Rest der Formel CO-Ar, CO-OAlk, CO-OAr, SO₂-Ar, SO₂-Alk, SO₂-OAr, 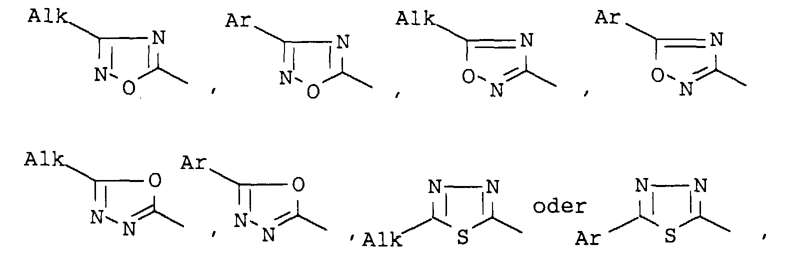
Q für Hydroxy oder eine unter alkalischen Reaktionsbedingungen abspaltbare Gruppe und
G für Phenyl, das durch C₁-C₄-Alkyl, Carboxyl, Hydroxysulfonyl oder C₁-C₄-Alkanoylamino substituiert sein kann, oder Naphthyl, das durch Hydroxysulfonyl substituiert sein kann, stehen.

2. Polyazofarbstoffe nach Anspruch 1, dadurch gekennzeichnet, daß B ein Brückenglied der Formel -SO₂-NH-A¹-NH-SO₂-, -SO₂-N(Alk)-A¹-NH-SO₂- oder -SO₂-N(Alk)-A¹-N(Alk)-SO₂- bedeutet, wobei Alk und A¹ jeweils die in Anspruch 1 genannte Bedeutung besitzen.

3. Polyazofarbstoffe nach den Ansprüchen 1 oder 2, dadurch gekennzeichnet, daß A¹ für m- oder p-Phenylen steht, das mit Hydroxysulfonyl substituiert sein kann.

4. Polyazofarbstoffe nach den Ansprüchen 1 bis 3, dadurch gekennzeichnet, daß p und q für die Zahl 0 stehen.

5. Polyazofarbstoffe nach den Ansprüchen 1 bis 4, dadurch gekennzeichnet, daß das Brückenglied B jeweils am Phenylring in para-Position zur Diazogruppe anknüpft.

6. Polyazofarbstoffe nach den Ansprüchen 1 bis 5, dadurch gekennzeichnet, daß sie der allgemeinen Formel II 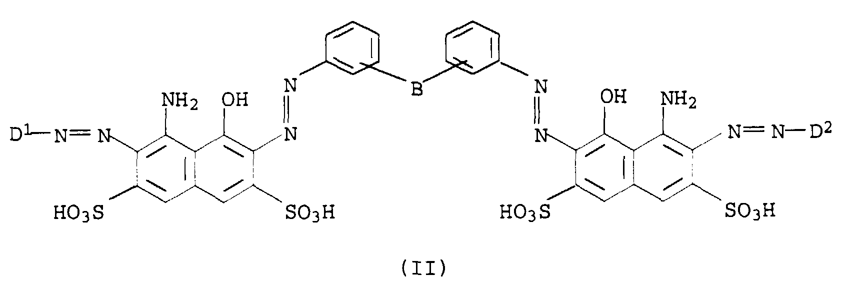 gehorchen, wobei B, D¹ und D² jeweils die in Anspruch 1 genannte Bedeutung besitzen.

7. Polyazofarbstoffe nach den Ansprüchen 1 bis 6, dadurch gekennzeichnet, daß Alk für C₁-C₆-Alkyl, das durch 1 oder 2 Sauerstoffatome in Etherfunktion oder 1 Sulfonylgruppe unterbrochen sein kann und durch Hydroxy, C₁-C₄-Alkanoyloxy, Sulfato, Chlor, Cyano, Carboxy, Hydroxysulfonyl, Phenyl oder Hydroxysulfonylphenyl substituiert sein kann, steht.

8. Polyazofarbstoffe nach den Ansprüchen 1 bis 7, dadurch gekennzeichnet, daß Ar für Phenyl, das durch C₁-C₄-Alkoxy, Carboxy, C₁-C₄-Alkanoylamino, Methoxyacetylamino, Hydroxysulfonyl oder einen Rest der Formel SO₂-CH=CH₂, SO₂-C₂H₄-Q, SO₂-NHAlk, SO₂-N(Alk)₂, SO₂-G, SO₂-OG, SO₂-NHG oder SO₂-N(Alk)G substituiert sein kann, steht, wobei Q, Alk und G die in Anspruch 1 genannte Bedeutung besitzen.

9. Polyazofarbstoffe nach den Ansprüchen 1 bis 8, dadurch gekennzeichnet, daß D¹ und D² für einen Rest der Formel 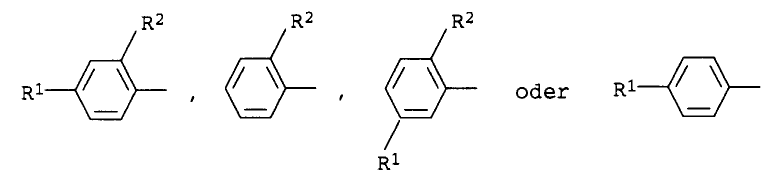 stehen, wobei R¹ und R² jeweils die in Anspruch 1 genannte Bedeutung besitzen.

10. Verwendung der Polyazofarbstoffe gemäß den Ansprüchen 1 bis 9 zum Färben von natürlichen oder synthetischen Substraten.

11. Sulfonamide der Formel VIIa 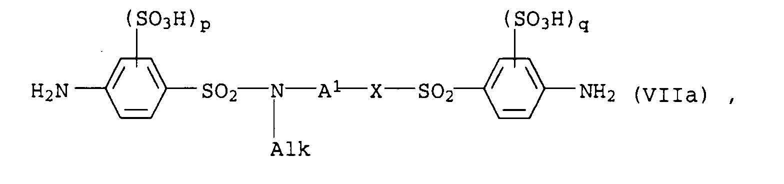 in der
p die Zahl 0 oder 1,
q die Zahl 0 oder 1,
A¹ Phenylen, das mit Hydroxysulfonyl substituiert sein kann,
X Sauerstoff, Imino oder einen Rest 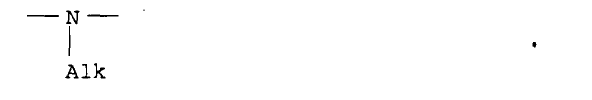 und
Alk C₁-C₈-Alkyl, das durch 1 bis 3 Sauerstoffatome in Etherfunktion oder durch 1 Schwefelatom oder 1 Sulfonylgruppe unterbrochen sein kann und durch Hydroxy, C₁-C₄-Alkanoyloxy, Benzoyloxy, Sulfato, Halogen, Cyano, Carbamoyl, Carboxy, Hydroxysulfonyl, Phenyl oder Hydroxysulfonylphenyl substituiert sein kann, oder C₅-C₈-Cycloalkyl
bedeuten.

12. Sulfonamide der Formel VIIb 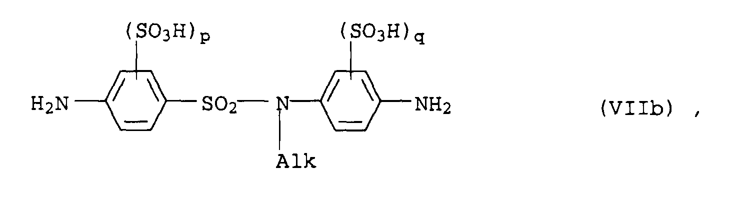 in der
p die Zahl 0 oder 1,
q die Zahl 0 oder 1 und
Alk C₁-C₈-Alkyl, das durch 1 bis 3 Sauerstoffatome in Etherfunktion oder durch 1 Schwefelatom oder 1 Sulfonylgruppe unterbrochen sein kann und durch Hydroxy, C₁-C₄-Alkanoyloxy, Benzoyloxy, Sulfato, Halogen, Cyano, Carbamoyl, Carboxy, Hydroxysulfonyl, Phenyl oder Hydroxysulfonylphenyl substituiert sein kann, oder C₅-C₈-Cycloalkyl
bedeuten.
